# EUROPÄISCHE PATENTANMELDUNG

(11) **EP 2 722 669 A1**
(43) Veröffentlichungstag der Anmeldung: **23.04.2014**
(21) Anmeldenummer: 12189463.8
(22) Anmeldetag: 22.10.2012
(51) Int. Cl.: G01N 33/52, G01N 33/543, G01N 33/94

(54) **Verfahren und Vorrichtung zum Nachweis von illegalen Drogen**

(71) Anmelder: Securetec Detektions-Systeme AG, 85649 Brunnthal (DE)
(72) Erfinder: Klaus, Sebastian, 82061 Neuried (DE); Schwieger, Frank, 82024 Taufkirchen (DE); Zimmermann, Verena, 81545 München (DE)
(74) Vertreter: Weiss, Wolfgang

(57) **Zusammenfassung**

Die vorliegende Erfindung betrifft ein Verfahren zur Bestimmung von Analyten, insbesondere illegalen Drogen, in einer Probe, sowie Testelemente, Probennahmeelemente und Kits, welche zur Durchführung des Verfahrens geeignet sind.

SEQUENCE LISTING

<110>  Securetec Detektions-Systeme AG

<120>  Verfahren und Vorrichtung zum Nachweis von illegalen Drogen

<130>  53936P EP

<160>  8

<170>  PatentIn version 3.3

<210>  1
<211>  5732
<212>  DNA
<213>  Homo sapiens

<220>
<221>  misc_feature
<222>  (1)..(5732)
<223>  Nucleotide sequence of human cannabinoid receptor 1

<400>  1
gcgccggcgc cgcctcccgc acgctactcc ctctgccacc ccttccttct ccacttcttt      60

tccgcctccg cctcttcttg tctcccgcgg cgccagcgcc ttcccttggc ccgggcgggg     120

gcctcggctc cctgcagagc tctccgtagt cagtggggga tatttcgttc tagcggacaa     180

ccagcccctg agctgggcga gaggtgccaa gggagcttct gtcccgagga ccaggggatg     240

cgaagggatt gccccctgtg ggtcactttc tcagtcattt tgagctcagc ctaatcaaag     300

actgaggtta tgaagtcgat cctagatggc cttgcagata ccaccttccg caccatcacc     360

actgacctcc tgtacgtggg ctcaaatgac attcagtacg aagacatcaa aggtgacatg     420

gcatccaaat tagggtactt cccacagaaa ttccctttaa cttcctttag gggaagtccc     480

ttccaagaga agatgactgc gggagacaac ccccagctag tcccagcaga ccaggtgaac     540

attacagaat tttacaacaa gtctctctcg tccttcaagg agaatgagga gaacatccag     600

tgtggggaga acttcatgga catagagtgt ttcatggtcc tgaaccccag ccagcagctg     660

gccattgcag tcctgtccct cacgctgggc accttcacgg tcctggagaa cctcctggtg     720

ctgtgcgtca tcctccactc ccgcagcctc cgctgcaggc cttcctacca cttcatcggc     780

agcctggcgg tggcagacct cctggggagt gtcatttttg tctacagctt cattgacttc     840

cacgtgttcc accgcaaaga tagccgcaac gtgtttctgt tcaaactggg tggggtcacg     900

gcctccttca ctgcctccgt gggcagcctg ttcctcacag ccatcgacag gtacatatcc     960

attcacaggc ccctggccta taagaggatt gtcaccaggc ccaaggccgt ggtggcgttt    1020

tgcctgatgt ggaccatagc cattgtgatc gccgtgctgc ctctcctggg ctggaactgc    1080

gagaaactgc aatctgtttg ctcagacatt ttcccacaca ttgatgaaac ctacctgatg    1140

ttctggatcg gggtcaccag cgtactgctt ctgttcatcg tgtatgcgta catgtatatt    1200

ctctggaagg ctcacagcca cgccgtccgc atgattcagc gtggcaccca gaagagcatc    1260

atcatccaca cgtctgagga tgggaaggta caggtgaccc ggccagacca agcccgcatg 1320
gacattaggt tagccaagac cctggtcctg atcctggtgg tgttgatcat ctgctggggc 1380
cctctgcttg caatcatggt gtatgatgtc tttgggaaga tgaacaagct cattaagacg 1440
gtgtttgcat tctgcagtat gctctgcctg ctgaactcca ccgtgaaccc catcatctat 1500
gctctgagga gtaaggacct gcgacacgct ttccggagca tgtttccctc ttgtgaaggc 1560
actgcgcagc ctctggataa cagcatgggg gactcggact gcctgcacaa acacgcaaac 1620
aatgcagcca gtgttcacag ggccgcagaa agctgcatca agagcacggt caagattgcc 1680
aaggtaacca tgtctgtgtc cacagacacg tctgccgagg ctctgtgagc ctgatgcctc 1740
cctggcagca caggaaaaga attttttttt ttaagctcaa aatctagaag agtctattgt 1800
ctccttggtt atattttttt aactttacca tgctcaatga aaaggtgatt gtcaccatga 1860
tcacttatca gtttgctaat gtttccatag tttaggtact caaactccat tctccagggg 1920
tttacagtga agaaagcctg ttgtttaagt gactgaacga tccttcaaag tctcaatgaa 1980
ataggaggga aacctttggc tacacaattg gaagtctaag aacccatgga aaaatgccat 2040
caaatgaata atgcctttgt aaccacaact ttcactataa tgtgaaatgt aactgtccgt 2100
agtatcagag atgtccattt ttacaagtta tagtactaga gatattttgt aaaatgtatt 2160
atgtcctgtg agatgtgtat cagtgtttat gtgctattaa tatttgttta gttcagcaaa 2220
actgaaaggt agacttttat gagaacaatg gacaagcagt ggatacgtgt caatgtgtgc 2280
actttttttc tatattattg cccatgatat aactttagaa ataaacctta atatttcttc 2340
aaatatctct atttaatttt gacactgaaa taaccgtaaa ggtttatttt tctgttacct 2400
caacaagaag aatttgaaga cttcaaaata ttgagcagaa ttcattcata cttaaaaatt 2460
tattagccct gcattttcat aggaagacac attatcttct ggactatagc tgttctaatg 2520
gattataatc agaatggaag agagaaagca tattgacttt ttttgagcga catctctgac 2580
tttctttagt ctttagctat tactggatct cttaagacag catgtgttaa tcttaatgta 2640
tatcgttatc actgtgcagt tgctgtttac ttgaatagta ttgtgttcct atattccagg 2700
tttaagtaga tttcatgcct gggtggccaa acaacagtct tcattttttt taattgaaaa 2760
gaagtagtgt ctggatcagt aaaattatac tgtgtgtgag tgtgaatata aatgtgtgta 2820
tgtgtgtttc tgtcctgtaa ctgttacagt aatgtcataa agtgagaaaa ctgtgaccaa 2880
gtataaactt ttaccacttg ctgcactctt gcacatggat tcagtttcta aaattgagtt 2940
cttcctgtaa tcttgttgat aaaaatactg actccaacca ttcaaaaatt tcaccccatc 3000
cctccttaag agattggatc aagtattact aaattgacct ttaggtatta cacaagacca 3060
gtgcttagca aaaaataatg acaggcatcc aaggaaggga tgtatttgta gtgttattgc 3120
caggaaagga gagtactttg gtttctgagc accgaatatt gagcaatatg tcagtcacta 3180

aaaggaagac agttctacag aaaaacaatg gtaacatttt tcaatagcgt gtgtagatag 3240

tatgcactat atacatcacg ttaaagtagg actatcacac ccagcccatg tggctaaaaa 3300

agctgaatca gacagtggat gagacacaca acggcagtga agaaccgata cacttggcat 3360

tgacgtctag ctatgctgta tctgtgcttt gcccacatgc ccttggtgac agctgagcac 3420

ccagctctgt cttggtaggt ttgggctaag gaacaaatct ctcctttgct cgtggttagc 3480

aagatacact caagcatgaa gataaacaca gctgctttct tcttacaccc cggtctcatg 3540

ctccttaatg gcgccatggg tgcttgttgg gcctttttcc agtaaggaat gatattgctg 3600

aagaatctac ttaaccctga caaattttaa ttataatctc ttcttataca gataaaacat 3660

gactcctaca aggccccaag gtttacatag tctgaagtga agtacagagc tggcatctat 3720

ctggtgattt ctagctctcg agatacccaa gcagcctgat ggggcagttc cccttcttac 3780

ggttcacgct ctaaggcagg atgtggctta tgagatactt tgcattgtct gtctgcacac 3840

cttgaatctg cctgctggct cccttacttt acctctctgt catgtgcaga tgaaggctca 3900

gggtgctaga ggattagtaa gatctctttc taaagacagg agagattatt tacaagaaga 3960

actcaccagg gtttagtttg catttaagaa ttgccagtct tttgtcctgc atcatcttga 4020

acattaatcc acatgtttca gagctcacca ggcagtacca atgctctttt cacagctatg 4080

aagagctaga gaaattcttg ttatggtaga aaaatttcac gattcatttt tgaaactgca 4140

tttgtgcgta tgcagtgtag attttatagt gtgttgtgct ttcaagatct aaatcatata 4200

taataaatta agggacaatg gggctgacag cactaaactt ggtgcttatt gatattctaa 4260

gaaatatctg tgaaatatca tcacgtatgt tatacaacct tcatttaaaa aggtttaaaa 4320

ctagttagat tcactttgac acttttcata tcatttctta acccaagtga cgaaaacatt 4380

gtccccaatg aatatactca ttagaattac catttgttaa tatcactcat taattaaccc 4440

cataattaga tccattaatt taaatgattt aaatttaagt aagttttata aggtctgaca 4500

tcagaggtat cttactttcc tctgaggatg atgtacttgc cctgaccatg cattttacca 4560

tcacacatgt tcagaaaggg ccaaattccc aacctgctca tttttttttt atcagagtca 4620

tgatgaatca gtcctagaat gtttcatttg cacaagtagg gctgcctcca agaggaacct 4680

ctgatttatt ttgtatgaaa tatatgtgaa aggatatgaa tctgagagat gctgtagaca 4740

tctgtcctac acttgagatg atttccaagc ctctctggca ctttgagtta agtctatctg 4800

gtattaaatg ccaaggacct tttgctgcct aaatccactc tgcaggaaat aggcccaacc 4860

accagatgag aattaggccc tggatgagta gcgctatagt tactgtcctg ttgattaatt 4920

tctgccattt catgtccata aaagagacca cccatatcat gcacacaatt agatttctca 4980

cactctaact gtatatttgt atgatatttt aaaatctcct aaatgctggg caatggctat 5040

taacaattaa ttgtcttgca ctggccttct gatgaaatgt taacaatgcc tattgtaata 5100

tagaaaaaaa cattctatct actgatttgg gctgaatgta tgtaaatagg tttctaaaaa 5160

gtcagatgtt tgagcagtgg cctacaaatc agtaattttc ggatgggaga gtttctttac 5220

attgccgtgg catcttaaaa gctatcttca tgtaaattga ctgtactagg cctactgggg 5280

atcagagttc ccaagaaagg aaaccttttc ttgtatctgg attcaaattt atttccaatg 5340

tttcaagcgg gaaacatgac tctttattgt ctgtaaatct aacattatta cttttcctct 5400

tagaagaata ttgtattgtt agatgtttgt tgagctggta acatcgttgc aaccactgca 5460

atatcttcgt tagtaatctg tataatactt tgtatacaag tactggtaag attgttatta 5520

aatgtagctt cagtcattaa attactatag caaagtagta cttcttctgt aatatttaca 5580

atgtattaag cccacagtat attttatttc aatgtaatta aactgttaac ttattcaaag 5640

agaaaacatc tcatcatgtc tattgtccaa agttacctgg aatcaaataa aaattctaga 5700

ttaccatgaa gaacataaaa aaaaaaaaaa aa 5732

<210> 2
<211> 472
<212> PRT
<213> Homo sapiens

<220>
<221> PEPTIDE
<222> (1)..(472)
<223> Amino acid sequence of human cannabinoid receptor 1

<400> 2

Met Lys Ser Ile Leu Asp Gly Leu Ala Asp Thr Thr Phe Arg Thr Ile
1               5                   10                  15

Thr Thr Asp Leu Leu Tyr Val Gly Ser Asn Asp Ile Gln Tyr Glu Asp
            20                  25                  30

Ile Lys Gly Asp Met Ala Ser Lys Leu Gly Tyr Phe Pro Gln Lys Phe
        35                  40                  45

Pro Leu Thr Ser Phe Arg Gly Ser Pro Phe Gln Glu Lys Met Thr Ala
    50                  55                  60

Gly Asp Asn Pro Gln Leu Val Pro Ala Asp Gln Val Asn Ile Thr Glu
65                  70                  75                  80

Phe Tyr Asn Lys Ser Leu Ser Ser Phe Lys Glu Asn Glu Glu Asn Ile
                85                  90                  95

Gln Cys Gly Glu Asn Phe Met Asp Ile Glu Cys Phe Met Val Leu Asn
            100                 105                 110

Pro Ser Gln Gln Leu Ala Ile Ala Val Leu Ser Leu Thr Leu Gly Thr
        115                 120                 125

Phe Thr Val Leu Glu Asn Leu Leu Val Leu Cys Val Ile Leu His Ser
    130                 135                 140

Arg Ser Leu Arg Cys Arg Pro Ser Tyr His Phe Ile Gly Ser Leu Ala
145                 150                 155                 160

Val Ala Asp Leu Leu Gly Ser Val Ile Phe Val Tyr Ser Phe Ile Asp
                165                 170                 175

Phe His Val Phe His Arg Lys Asp Ser Arg Asn Val Phe Leu Phe Lys
            180                 185                 190

Leu Gly Gly Val Thr Ala Ser Phe Thr Ala Ser Val Gly Ser Leu Phe
        195                 200                 205

Leu Thr Ala Ile Asp Arg Tyr Ile Ser Ile His Arg Pro Leu Ala Tyr
    210                 215                 220

Lys Arg Ile Val Thr Arg Pro Lys Ala Val Val Ala Phe Cys Leu Met
225                 230                 235                 240

Trp Thr Ile Ala Ile Val Ile Ala Val Leu Pro Leu Leu Gly Trp Asn
                245                 250                 255

Cys Glu Lys Leu Gln Ser Val Cys Ser Asp Ile Phe Pro His Ile Asp
            260                 265                 270

Glu Thr Tyr Leu Met Phe Trp Ile Gly Val Thr Ser Val Leu Leu Leu
        275                 280                 285

Phe Ile Val Tyr Ala Tyr Met Tyr Ile Leu Trp Lys Ala His Ser His
    290                 295                 300

Ala Val Arg Met Ile Gln Arg Gly Thr Gln Lys Ser Ile Ile Ile His
305                 310                 315                 320

Thr Ser Glu Asp Gly Lys Val Gln Val Thr Arg Pro Asp Gln Ala Arg
                325                 330                 335

Met Asp Ile Arg Leu Ala Lys Thr Leu Val Leu Ile Leu Val Val Leu
            340                 345                 350

Ile Ile Cys Trp Gly Pro Leu Leu Ala Ile Met Val Tyr Asp Val Phe
        355                 360                 365

Gly Lys Met Asn Lys Leu Ile Lys Thr Val Phe Ala Phe Cys Ser Met
    370                 375                 380

Leu Cys Leu Leu Asn Ser Thr Val Asn Pro Ile Ile Tyr Ala Leu Arg
385                 390                 395                 400

Ser Lys Asp Leu Arg His Ala Phe Arg Ser Met Phe Pro Ser Cys Glu
                405                 410                 415

Gly Thr Ala Gln Pro Leu Asp Asn Ser Met Gly Asp Ser Asp Cys Leu
            420                 425                 430

His Lys His Ala Asn Asn Ala Ala Ser Val His Arg Ala Ala Glu Ser
        435                 440                 445

Cys Ile Lys Ser Thr Val Lys Ile Ala Lys Val Thr Met Ser Val Ser
    450                 455                 460

Thr Asp Thr Ser Ala Glu Ala Leu
465                 470

<210>  3
<211>  5387
<212>  DNA
<213>  Homo sapiens

<220>
<221>  misc_feature
<222>  (1)..(5387)
<223>  Nucleotide sequence of human cannabinoid receptor 2

<400>  3
gattgccccc tgtgggtcac tttctcagtc attttgagct cagcctaatc aaagactgag      60

gttatgaagt cgatcctaga tggccttgca gataccacct tccgcaccat caccactgac     120

ctcctgggaa gtcccttcca agagaagatg actgcgggag acaaccccca gctagtccca     180

gcagaccagg tgaacattac agaattttac aacaagtctc tctcgtcctt caaggagaat     240

gaggagaaca tccagtgtgg ggagaacttc atggacatag agtgtttcat ggtcctgaac     300

cccagccagc agctggccat tgcagtcctg tccctcacgc tgggcacctt cacggtcctg     360

gagaacctcc tggtgctgtg cgtcatcctc cactcccgca gcctccgctg caggccttcc     420

taccacttca tcggcagcct ggcggtggca gacctcctgg ggagtgtcat ttttgtctac     480

agcttcattg acttccacgt gttccaccgc aaagatagcc gcaacgtgtt tctgttcaaa     540

ctgggtgggg tcacggcctc cttcactgcc tccgtgggca gcctgttcct cacagccatc     600

gacaggtaca tatccattca caggcccctg gcctataaga ggattgtcac caggcccaag     660

gccgtggtgg cgttttgcct gatgtggacc atagccattg tgatcgccgt gctgcctctc     720

ctgggctgga actgcgagaa actgcaatct gtttgctcag acattttccc acacattgat     780

gaaacctacc tgatgttctg gatcggggtc accagcgtac tgcttctgtt catcgtgtat     840

gcgtacatgt atattctctg gaaggctcac agccacgccg tccgcatgat tcagcgtggc 900

acccagaaga gcatcatcat ccacacgtct gaggatggga aggtacaggt gacccggcca 960

gaccaagccc gcatggacat taggttagcc aagaccctgg tcctgatcct ggtggtgttg 1020

atcatctgct ggggccctct gcttgcaatc atggtgtatg atgtctttgg gaagatgaac 1080

aagctcatta agacggtgtt tgcattctgc agtatgctct gcctgctgaa ctccaccgtg 1140

aaccccatca tctatgctct gaggagtaag gacctgcgac acgctttccg gagcatgttt 1200

ccctcttgtg aaggcactgc gcagcctctg gataacagca tgggggactc ggactgcctg 1260

cacaaacacg caaacaatgc agccagtgtt cacagggccg cagaaagctg catcaagagc 1320

acggtcaaga ttgccaaggt aaccatgtct gtgtccacag acacgtctgc cgaggctctg 1380

tgagcctgat gcctccctgg cagcacagga aaagaatttt tttttttaag ctcaaaatct 1440

agaagagtct attgtctcct tggttatatt tttttaactt taccatgctc aatgaaaagg 1500

tgattgtcac catgatcact tatcagtttg ctaatgtttc catagtttag gtactcaaac 1560

tccattctcc aggggtttac agtgaagaaa gcctgttgtt taagtgactg aacgatcctt 1620

caaagtctca atgaaatagg agggaaacct ttggctacac aattggaagt ctaagaaccc 1680

atggaaaaat gccatcaaat gaataatgcc tttgtaacca caactttcac tataatgtga 1740

aatgtaactg tccgtagtat cagagatgtc catttttaca agttatagta ctagagatat 1800

tttgtaaaat gtattatgtc ctgtgagatg tgtatcagtg tttatgtgct attaatattt 1860

gtttagttca gcaaaactga aaggtagact tttatgagaa caatggacaa gcagtggata 1920

cgtgtcaatg tgtgcacttt ttttctatat tattgcccat gatataactt tagaaataaa 1980

ccttaatatt tcttcaaata tctctattta attttgacac tgaaataacc gtaaaggttt 2040

atttttctgt tacctcaaca agaagaattt gaagacttca aaatattgag cagaattcat 2100

tcatacttaa aaatttatta gccctgcatt ttcataggaa gacacattat cttctggact 2160

atagctgttc taatggatta taatcagaat ggaagagaga aagcatattg actttttttg 2220

agcgacatct ctgactttct ttagtcttta gctattactg gatctcttaa gacagcatgt 2280

gttaatctta atgtatatcg ttatcactgt gcagttgctg tttacttgaa tagtattgtg 2340

ttcctatatt ccaggtttaa gtagatttca tgcctgggtg gccaaacaac agtcttcatt 2400

ttttttaatt gaaaagaagt agtgtctgga tcagtaaaat tatactgtgt gtgagtgtga 2460

atataaatgt gtgtatgtgt gtttctgtcc tgtaactgtt acagtaatgt cataaagtga 2520

gaaaactgtg accaagtata aacttttacc acttgctgca ctcttgcaca tggattcagt 2580

ttctaaaatt gagttcttcc tgtaatcttg ttgataaaaa tactgactcc aaccattcaa 2640

aaatttcacc ccatccctcc ttaagagatt ggatcaagta ttactaaatt gacctttagg 2700

tattacacaa gaccagtgct tagcaaaaaa taatgacagg catccaagga agggatgtat 2760

ttgtagtgtt attgccagga aaggagagta ctttggtttc tgagcaccga atattgagca 2820

atatgtcagt cactaaaagg aagacagttc tacagaaaaa caatggtaac atttttcaat 2880

agcgtgtgta gatagtatgc actatataca tcacgttaaa gtaggactat cacacccagc 2940

ccatgtggct aaaaaagctg aatcagacag tggatgagac acacaacggc agtgaagaac 3000

cgatacactt ggcattgacg tctagctatg ctgtatctgt gctttgccca catgcccttg 3060

gtgacagctg agcacccagc tctgtcttgg taggtttggg ctaaggaaca aatctctcct 3120

ttgctcgtgg ttagcaagat acactcaagc atgaagataa acacagctgc tttcttctta 3180

caccccggtc tcatgctcct taatggcgcc atgggtgctt gttgggcctt tttccagtaa 3240

ggaatgatat tgctgaagaa tctacttaac cctgacaaat tttaattata atctcttctt 3300

atacagataa aacatgactc ctacaaggcc ccaaggttta catagtctga agtgaagtac 3360

agagctggca tctatctggt gatttctagc tctcgagata cccaagcagc ctgatggggc 3420

agttcccctt cttacggttc acgctctaag gcaggatgtg gcttatgaga tactttgcat 3480

tgtctgtctg cacaccttga atctgcctgc tggctccctt actttacctc tctgtcatgt 3540

gcagatgaag gctcagggtg ctagaggatt agtaagatct ctttctaaag acaggagaga 3600

ttatttacaa gaagaactca ccagggttta gtttgcattt aagaattgcc agtcttttgt 3660

cctgcatcat cttgaacatt aatccacatg tttcagagct caccaggcag taccaatgct 3720

cttttcacag ctatgaagag ctagagaaat tcttgttatg gtagaaaaat ttcacgattc 3780

atttttgaaa ctgcatttgt gcgtatgcag tgtagatttt atagtgtgtt gtgctttcaa 3840

gatctaaatc atatataata aattaaggga caatggggct gacagcacta aacttggtgc 3900

ttattgatat tctaagaaat atctgtgaaa tatcatcacg tatgttatac aaccttcatt 3960

taaaaaggtt taaaactagt tagattcact ttgacacttt tcatatcatt tcttaaccca 4020

agtgacgaaa acattgtccc caatgaatat actcattaga attaccattt gttaatatca 4080

ctcattaatt aaccccataa ttagatccat taatttaaat gatttaaatt taagtaagtt 4140

ttataaggtc tgacatcaga ggtatcttac tttcctctga ggatgatgta cttgccctga 4200

ccatgcattt taccatcaca catgttcaga aagggccaaa ttcccaacct gctcattttt 4260

tttttatcag agtcatgatg aatcagtcct agaatgtttc atttgcacaa gtagggctgc 4320

ctccaagagg aacctctgat ttattttgta tgaaatatat gtgaaaggat atgaatctga 4380

gagatgctgt agacatctgt cctacacttg agatgatttc caagcctctc tggcactttg 4440

agttaagtct atctggtatt aaatgccaag gaccttttgc tgcctaaatc cactctgcag 4500

gaaataggcc caaccaccag atgagaatta ggccctggat gagtagcgct atagttactg 4560

tcctgttgat taatttctgc catttcatgt ccataaaaga gaccacccat atcatgcaca 4620

caattagatt tctcacactc taactgtata tttgtatgat attttaaaat ctcctaaatg 4680

ctgggcaatg gctattaaca attaattgtc ttgcactggc cttctgatga aatgttaaca 4740

atgcctattg taatatagaa aaaaacattc tatctactga tttgggctga atgtatgtaa 4800

ataggtttct aaaaagtcag atgtttgagc agtggcctac aaatcagtaa ttttcggatg 4860

ggagagtttc tttacattgc cgtggcatct taaaagctat cttcatgtaa attgactgta 4920

ctaggcctac tggggatcag agttcccaag aaaggaaacc ttttcttgta tctggattca 4980

aatttatttc caatgtttca agcgggaaac atgactcttt attgtctgta aatctaacat 5040

tattactttt cctcttagaa gaatattgta ttgttagatg tttgttgagc tggtaacatc 5100

gttgcaacca ctgcaatatc ttcgttagta atctgtataa tactttgtat acaagtactg 5160

gtaagattgt tattaaatgt agcttcagtc attaaattac tatagcaaag tagtacttct 5220

tctgtaatat ttacaatgta ttaagcccac agtatatttt atttcaatgt aattaaactg 5280

ttaacttatt caaagagaaa acatctcatc atgtctattg tccaaagtta cctggaatca 5340

aataaaaatt ctagattacc atgaagaaca taaaaaaaaa aaaaaaa 5387

<210> 4
<211> 439
<212> PRT
<213> Homo sapiens

<220>
<221> PEPTIDE
<222> (1)..(439)
<223> Amino acid sequence of human cannabinoid receptor 2

<400> 4

Met Lys Ser Ile Leu Asp Gly Leu Ala Asp Thr Thr Phe Arg Thr Ile
1               5                   10                  15

Thr Thr Asp Leu Leu Gly Ser Pro Phe Gln Glu Lys Met Thr Ala Gly
            20                  25                  30

Asp Asn Pro Gln Leu Val Pro Ala Asp Gln Val Asn Ile Thr Glu Phe
        35                  40                  45

Tyr Asn Lys Ser Leu Ser Ser Phe Lys Glu Asn Glu Glu Asn Ile Gln
    50                  55                  60

Cys Gly Glu Asn Phe Met Asp Ile Glu Cys Phe Met Val Leu Asn Pro
65                  70                  75                  80

Ser Gln Gln Leu Ala Ile Ala Val Leu Ser Leu Thr Leu Gly Thr Phe
                85                  90                  95

Thr Val Leu Glu Asn Leu Leu Val Leu Cys Val Ile Leu His Ser Arg
            100                 105                 110

Ser Leu Arg Cys Arg Pro Ser Tyr His Phe Ile Gly Ser Leu Ala Val

115                 120                 125

Ala Asp Leu Leu Gly Ser Val Ile Phe Val Tyr Ser Phe Ile Asp Phe
    130                 135                 140

His Val Phe His Arg Lys Asp Ser Arg Asn Val Phe Leu Phe Lys Leu
145                 150                 155                 160

Gly Gly Val Thr Ala Ser Phe Thr Ala Ser Val Gly Ser Leu Phe Leu
                165                 170                 175

Thr Ala Ile Asp Arg Tyr Ile Ser Ile His Arg Pro Leu Ala Tyr Lys
            180                 185                 190

Arg Ile Val Thr Arg Pro Lys Ala Val Val Ala Phe Cys Leu Met Trp
        195                 200                 205

Thr Ile Ala Ile Val Ile Ala Val Leu Pro Leu Leu Gly Trp Asn Cys
    210                 215                 220

Glu Lys Leu Gln Ser Val Cys Ser Asp Ile Phe Pro His Ile Asp Glu
225                 230                 235                 240

Thr Tyr Leu Met Phe Trp Ile Gly Val Thr Ser Val Leu Leu Leu Phe
                245                 250                 255

Ile Val Tyr Ala Tyr Met Tyr Ile Leu Trp Lys Ala His Ser His Ala
            260                 265                 270

Val Arg Met Ile Gln Arg Gly Thr Gln Lys Ser Ile Ile Ile His Thr
        275                 280                 285

Ser Glu Asp Gly Lys Val Gln Val Thr Arg Pro Asp Gln Ala Arg Met
    290                 295                 300

Asp Ile Arg Leu Ala Lys Thr Leu Val Leu Ile Leu Val Val Leu Ile
305                 310                 315                 320

Ile Cys Trp Gly Pro Leu Leu Ala Ile Met Val Tyr Asp Val Phe Gly
                325                 330                 335

Lys Met Asn Lys Leu Ile Lys Thr Val Phe Ala Phe Cys Ser Met Leu
            340                 345                 350

Cys Leu Leu Asn Ser Thr Val Asn Pro Ile Ile Tyr Ala Leu Arg Ser
        355                 360                 365

Lys Asp Leu Arg His Ala Phe Arg Ser Met Phe Pro Ser Cys Glu Gly
    370                 375                 380

Thr Ala Gln Pro Leu Asp Asn Ser Met Gly Asp Ser Asp Cys Leu His
385                 390                 395                 400

Lys His Ala Asn Asn Ala Ala Ser Val His Arg Ala Ala Glu Ser Cys
                405                 410                 415

Ile Lys Ser Thr Val Lys Ile Ala Lys Val Thr Met Ser Val Ser Thr
            420                 425                 430

Asp Thr Ser Ala Glu Ala Leu
        435

<210>  5
<211>  4959
<212>  DNA
<213>  Homo sapiens

<220>
<221>  misc_feature
<222>  (1)..(4959)
<223>  Nucleotide sequence of human opioid receptor k1

<400>  5
agtgggagac gtgcgctgag aggcgggggc tgcgctcggc ggaacagcag ccctcgggcg      60

gagagcgggg ccggggtccg agagcaggtg atgccaagag ctgagcggga ctcgtgagcg     120

cgcggttcag cacctaccag ggcgtcccgt aaaaaacctc gccttcgcct gtctctggga     180

accatagcgc cgcaggtgcc gcctgtcctc gccttcctgc tgcaatcgcc ccaccatgga     240

ctccccgatc cagatcttcc gcggggagcc gggccctacc tgcgccccga gcgcctgcct     300

gccccccaac agcagcgcct ggtttcccgg ctgggccgag cccgacagca acggcagcgc     360

cggctcggag gacgcgcagc tggagcccgc gcacatctcc ccggccatcc cggtcatcat     420

cacggcggtc tactccgtag tgttcgtcgt gggcttggtg ggcaactcgc tggtcatgtt     480

cgtgatcatc cgatacacaa agatgaagac agcaaccaac atttacatat ttaacctggc     540

tttggcagat gctttagtta ctacaaccat gccctttcag agtacggtct acttgatgaa     600

ttcctggcct tttggggatg tgctgtgcaa gatagtaatt tccattgatt actacaacat     660

gttcaccagc atcttcacct tgaccatgat gagcgtggac cgctacattg ccgtgtgcca     720

ccccgtgaag gctttggact tccgcacacc cttgaaggca aagatcatca atatctgcat     780

ctggctgctg tcgtcatctg ttggcatctc tgcaatagtc cttggaggca ccaaagtcag     840

ggaagacgtc gatgtcattg agtgctcctt gcagttccca gatgatgact actcctggtg     900

ggacctcttc atgaagatct gcgtcttcat ctttgccttc gtgatccctg tcctcatcat     960

catcgtctgc tacaccctga tgatcctgcg tctcaagagc gtccggctcc tttctggctc    1020

ccgagagaaa gatcgcaacc tgcgtaggat caccagactg gtcctggtgg tggtggcagt    1080

cttcgtcgtc tgctggactc ccattcacat attcatcctg gtggaggctc tggggagcac 1140
ctcccacagc acagctgctc tctccagcta ttacttctgc atcgccttag gctataccaa 1200
cagtagcctg aatcccattc tctacgcctt tcttgatgaa aacttcaagc ggtgtttccg 1260
ggacttctgc tttccactga agatgaggat ggagcggcag agcactagca gagtccgaaa 1320
tacagttcag gatcctgctt acctgaggga catcgatggg atgaataaac cagtatgact 1380
agtcgtggag atgtcttcgt acagttcttc gggaagagag gagttcaatg atctaggttt 1440
aactcagatc actactgcag tctgacatga aaagatagaa tttaaaataa acttttagag 1500
atctcatggt ctgaagtcat cagatgcaga ccacgttcgt gatcagtgga aacatcaagg 1560
acaaaaggtc cacgcaacac atgcacctct tctctagggc acagaagcga ggcagagcct 1620
cagttcctct gatgaacaag ggaacaggtc ttttccttct ggtttgctag gtaaggttca 1680
gcacccatct gctgtggcct tcctatgaaa cgtagtttca aaagctcggt ttaagaaaaa 1740
aggaaaagaa aaaacattgc tttcagagct caatgggcat ccaatccaga tgtttcacaa 1800
tgacagcaac aacctccaac ggaatgggaa ctatttccag ggttcatgcc cagctcactg 1860
tatgctgtgt ggacatgcat ttcatgtggc tgtgtggtaa gaattacagc ttacatatgg 1920
cttggaccca catctgagga atctgatgtt cacttatacc agaacattat cttgctattt 1980
atgaaattat ttaaacgttc aaaagattgt ttttaacatg gtttaatttc ccaaaaacta 2040
cagttttttt tcttagcatg ctattcaggt aaacagtctt ataataaagc atgtcccatt 2100
gtcaagaaac ataaagtggt gtgaatacca ctgaaaatat atatatagta tcttctgtaa 2160
ataatagtac ctgtgtgaat aaggaatagg cttgcctccc agccaggcaa tttcctgagg 2220
gcacactaat gatatcccct gagttgctaa gttgatgctg agacattttg ctgggaatta 2280
gtcatggcat gatctctttc agactccctg aataccattt agtcccgtaa cagtgctcac 2340
aactcatgtg ctaatgaatc acaaaggctt taactagctc ccaggttgta gccttcgcag 2400
gatctagttt atttgccaca tctctttatg aacatatagc gattcgcaga tctctctatt 2460
cacggagagg aaggtgtttt gcttctgtag atctcaaggt actattttgt ggctctcagc 2520
aggaagtaga attgttccta aatgtgtgct gaatgaggac atgatgtccc tcctggtgcc 2580
aggacacatc ctgcatggca ttctgtgaag ggcatacctg ctgaggataa tgccaggagc 2640
agcacattta gggtaatttt gctaaaactt ccagatgaca tataaattcc ctctttttcc 2700
ccctgaaact taccatttca gaggtaatgg ctttttctgt aatttgcctg agaagaaaaa 2760
agggataatt tagaaataac actccaagcc tatatattac tagggctgca tcctctggaa 2820
tttaatagaa aaattgaata tatagcccta tgtaactaca gacatcatta caactgttaa 2880
gttctgttca ccatgcaagt tctcggtggt gtatgtactc tcaccttttg gaaacaaaac 2940
cttgcattga aggttttaca catggcttca gaatgttact ctgaccatgt ttgcctctac 3000

tccagattat catggcacct aacacagatg acatatggca gaatggaagt gatttctcct 3060
aatgaaaatg aagtgctctt ttgagtattt cttgttttat tgagattcct atctgccctt 3120
gtatttttat aactgaatgt aaataggaat tgcttaaata ctggtttaca ctaatcccat 3180
agttaaaaat cttggctatg gtacaggaag gatatctata tagaggatag ttccagaaac 3240
aagccctggt gaaataggaa atataaaaac ttctttatgg aacaggaaaa cccaagagac 3300
tttctcgtca gattcctttc cagtgcaaag tataacagag ataggattat gagatgaaat 3360
cacagtagtg tgtttccttt aatttttctt gtcttctttt tttttttttt tttttttgag 3420
acggagtctc gctctgttgc ccaggctgga gtgcggtagc acagtcttgg ctcactgcaa 3480
cctccacctc ctgggttcaa gcgattctcc tgcctctgcc tcctaagtag ctgggattac 3540
aggcgtgcac cgccaagccc ggctaatttt tgtattttta gtagagacgg ggtttcacca 3600
tgttatgcag gctggtctca aactcctgac ctcagaatat tcacctgcct cggcctccca 3660
aagtgctggg attacagatg tgaatggcca cgcctggcct gtttccttta agttagcact 3720
tcacgtgctc ttacagcgtt aattgccatt attggtttgc ctgattttcc tgcatctcct 3780
catatgtaag tctgcagccc aaggagagaa tagtagctgt atgtgcccac aaggggtgtc 3840
caagcttctt gattcttgtt aatatcgact ccccaacatt gaaaaaggag tgaagaaggg 3900
aatatcattt tagggtactg ctttaaagga atgataaagt aaacaacgta gcagggaaaa 3960
taccacttgc acaaatgtat ttctttgtca aagtgtgtac acgctttctg tttgtcctga 4020
tgtcctgctg tgaagcaggg accacattgc catttattca ttgttccagg caatgcattt 4080
gtccaaaccc ctctggctct ctgatatctc tcatttcttg atgttcctat tctctgacct 4140
caacaaggag gacactgtct gttgtgtctc tttgagacta gaactccatg tgtttaacct 4200
tcagatccat tctctacatg aaccctattc tttccaccct ggtaagaagg tctggaagac 4260
aggtcctaac gaggggtcag agaaggcaca gagaaatgaa ccagccatgc aggggcagca 4320
ctgaggagga aaccccccac tctaactggg aggagagtgg aaacactctt agtatctcag 4380
agtagttacc aatctaagag catccttcga gtttaagtca aaactctagg cttaatacca 4440
aaaataatac atcatttgga aaaatagctt ggagaaaaaa aatcaataaa gagtcacata 4500
aaatcttaag aaaatggttc tattctgtga tatattacta agaaatacag ttcttaaacg 4560
tgtataacta ttgtcagaca atttataggt gtttcatcta gtcctgggat gaaatacgat 4620
gactcatgca aattcaggga gcacactggc tgactttaaa ttggaacatt gtaaagtgaa 4680
agctgtgaaa ggttgtgtgt cctatcaatg agatttattt tctaaccaag agctgttcgg 4740
atgattcagt ataaatacga aaatcacatc caaaaacggt ataacccagc ttccttaagg 4800
caattttctt ctctgaaaca agaatatact catatgttct ttactataat gtatatgatt 4860
tttatcttgt actttaaaag atgataatta tgcattgtat atacgattgt gtgccttgca 4920
ataaaatcaa aactgtacct gctgaaaatc acaacagtc 4959

<210>  6
<211>  380
<212>  PRT
<213>  Homo sapiens

<220>
<221>  PEPTIDE
<222>  (1)..(380)
<223>  Amino acid sequence of human opioid receptor k1

<400>  6

Met Asp Ser Pro Ile Gln Ile Phe Arg Gly Glu Pro Gly Pro Thr Cys
1               5                   10                  15

Ala Pro Ser Ala Cys Leu Pro Pro Asn Ser Ser Ala Trp Phe Pro Gly
            20                  25                  30

Trp Ala Glu Pro Asp Ser Asn Gly Ser Ala Gly Ser Glu Asp Ala Gln
        35                  40                  45

Leu Glu Pro Ala His Ile Ser Pro Ala Ile Pro Val Ile Ile Thr Ala
    50                  55                  60

Val Tyr Ser Val Val Phe Val Val Gly Leu Val Gly Asn Ser Leu Val
65                  70                  75                  80

Met Phe Val Ile Ile Arg Tyr Thr Lys Met Lys Thr Ala Thr Asn Ile
                85                  90                  95

Tyr Ile Phe Asn Leu Ala Leu Ala Asp Ala Leu Val Thr Thr Thr Met
            100                 105                 110

Pro Phe Gln Ser Thr Val Tyr Leu Met Asn Ser Trp Pro Phe Gly Asp
        115                 120                 125

Val Leu Cys Lys Ile Val Ile Ser Ile Asp Tyr Tyr Asn Met Phe Thr
    130                 135                 140

Ser Ile Phe Thr Leu Thr Met Met Ser Val Asp Arg Tyr Ile Ala Val
145                 150                 155                 160

Cys His Pro Val Lys Ala Leu Asp Phe Arg Thr Pro Leu Lys Ala Lys
                165                 170                 175

Ile Ile Asn Ile Cys Ile Trp Leu Leu Ser Ser Ser Val Gly Ile Ser
            180                 185                 190

Ala Ile Val Leu Gly Gly Thr Lys Val Arg Glu Asp Val Asp Val Ile
        195                 200                 205

Glu Cys Ser Leu Gln Phe Pro Asp Asp Asp Tyr Ser Trp Trp Asp Leu
    210                 215                 220

Phe Met Lys Ile Cys Val Phe Ile Phe Ala Phe Val Ile Pro Val Leu
225                 230                 235                 240

Ile Ile Ile Val Cys Tyr Thr Leu Met Ile Leu Arg Leu Lys Ser Val
                245                 250                 255

Arg Leu Leu Ser Gly Ser Arg Glu Lys Asp Arg Asn Leu Arg Arg Ile
            260                 265                 270

Thr Arg Leu Val Leu Val Val Val Ala Val Phe Val Val Cys Trp Thr
        275                 280                 285

Pro Ile His Ile Phe Ile Leu Val Glu Ala Leu Gly Ser Thr Ser His
    290                 295                 300

Ser Thr Ala Ala Leu Ser Ser Tyr Tyr Phe Cys Ile Ala Leu Gly Tyr
305                 310                 315                 320

Thr Asn Ser Ser Leu Asn Pro Ile Leu Tyr Ala Phe Leu Asp Glu Asn
                325                 330                 335

Phe Lys Arg Cys Phe Arg Asp Phe Cys Phe Pro Leu Lys Met Arg Met
            340                 345                 350

Glu Arg Gln Ser Thr Ser Arg Val Arg Asn Thr Val Gln Asp Pro Ala
        355                 360                 365

Tyr Leu Arg Asp Ile Asp Gly Met Asn Lys Pro Val
    370                 375                 380

<210>  7
<211>  2178
<212>  DNA
<213>  Homo sapiens

<220>
<221>  misc_feature
<222>  (1)..(2178)
<223>  Nucleotide sequence of human opioid receptor µ1; transcript
       variant MOR-1

<400>  7
gatgagcctc tgtgaactac taaggtggga gggggctata cgcagaggag aatgtcagat      60

gctcagctcg gtcccctccg cctgacgctc ctctctgtct cagccaggac tggtttctgt     120

aagaaacagc aggagctgtg gcagcggcga aaggaagcgg ctgaggcgct tggaacccga     180

aaagtctcgg tgctcctggc tacctcgcac agcggtgccc gcccggccgt cagtaccatg 240
gacagcagcg ctgcccccac gaacgccagc aattgcactg atgccttggc gtactcaagt 300
tgctccccag cacccagccc cggttcctgg gtcaacttgt cccacttaga tggcaacctg 360
tccgacccat gcggtccgaa ccgcaccgac ctgggcggga gagacagcct gtgccctccg 420
accggcagtc cctccatgat cacggccatc acgatcatgg ccctctactc catcgtgtgc 480
gtggtggggc tcttcggaaa cttcctggtc atgtatgtga ttgtcagata caccaagatg 540
aagactgcca ccaacatcta cattttcaac cttgctctgg cagatgcctt agccaccagt 600
accctgccct tccagagtgt gaattaccta atgggaacat ggccatttgg aaccatcctt 660
tgcaagatag tgatctccat agattactat aacatgttca ccagcatatt caccctctgc 720
accatgagtg ttgatcgata cattgcagtc tgccaccctg tcaaggcctt agatttccgt 780
actccccgaa atgccaaaat tatcaatgtc tgcaactgga tcctctcttc agccattggt 840
cttcctgtaa tgttcatggc tacaacaaaa tacaggcaag gttccataga ttgtacacta 900
acattctctc atccaacctg gtactgggaa aacctgctga agatctgtgt tttcatcttc 960
gccttcatta tgccagtgct catcattacc gtgtgctatg gactgatgat cttgcgcctc 1020
aagagtgtcc gcatgctctc tggctccaaa gaaaaggaca ggaatcttcg aaggatcacc 1080
aggatggtgc tggtggtggt ggctgtgttc atcgtctgct ggactcccat tcacatttac 1140
gtcatcatta aagccttggt tacaatccca gaaactacgt tccagactgt ttcttggcac 1200
ttctgcattg ctctaggtta cacaaacagc tgcctcaacc cagtccttta tgcatttctg 1260
gatgaaaact tcaaacgatg cttcagagag ttctgtatcc caacctcttc caacattgag 1320
caacaaaact ccactcgaat tcgtcagaac actagagacc acccctccac ggccaataca 1380
gtggatagaa ctaatcatca gctagaaaat ctggaagcag aaactgctcc gttgccctaa 1440
cagggtctca tgccattccg accttcacca agcttagaag ccaccatgta tgtggaagca 1500
ggttgcttca agaatgtgta ggaggctcta attctctagg aaagtgcctg cttttaggtc 1560
atccaacctc tttcctctct ggccactctg ctctgcacat tagagggaca gccaaaagta 1620
agtggagcat ttggaaggaa aggaatatac cacaccgagg agtccagttt gtgcaagaca 1680
cccagtggaa ccaaaaccca tcgtggtatg tgaattgaag tcatcataaa aggtgaccct 1740
tctgtctgta agattttatt ttcaagcaaa tatttatgac ctcaacaaag aagaaccatc 1800
ttttgttaag ttcaccgtag taacacataa agtaaatgct acctctgatc aaagcacctt 1860
gaatggaagg tccgagtctt tttagtgttt tgcaagggaa tgaatccatt attctatttt 1920
agacttttaa cttcacctta aaattagcat ctggctaagg catcattttc acctccattt 1980
cttggttttg tattgtttaa aaaaataaca tctctttcat ctagctccat aattgcaagg 2040
gaagagatta gcatgaaagg taatctgaaa cacagtcatg tgtcagctgt agaaaggttg 2100
attctcatgc actgcaaata cttccaaaga gtcatcatgg gggatttttc attcttaggc 2160

tttcagtggt ttgttcct                                                    2178

<210>  8
<211>  400
<212>  PRT
<213>  Homo sapiens

<220>
<221>  PEPTIDE
<222>  (1)..(400)
<223>  Amino acid sequence of human opioid receptor μ1; transcript
       variant MOR-1

<400>  8

Met Asp Ser Ser Ala Ala Pro Thr Asn Ala Ser Asn Cys Thr Asp Ala
1               5                   10                  15

Leu Ala Tyr Ser Ser Cys Ser Pro Ala Pro Ser Pro Gly Ser Trp Val
            20                  25                  30

Asn Leu Ser His Leu Asp Gly Asn Leu Ser Asp Pro Cys Gly Pro Asn
        35                  40                  45

Arg Thr Asp Leu Gly Gly Arg Asp Ser Leu Cys Pro Pro Thr Gly Ser
    50                  55                  60

Pro Ser Met Ile Thr Ala Ile Thr Ile Met Ala Leu Tyr Ser Ile Val
65                  70                  75                  80

Cys Val Val Gly Leu Phe Gly Asn Phe Leu Val Met Tyr Val Ile Val
                85                  90                  95

Arg Tyr Thr Lys Met Lys Thr Ala Thr Asn Ile Tyr Ile Phe Asn Leu
            100                 105                 110

Ala Leu Ala Asp Ala Leu Ala Thr Ser Thr Leu Pro Phe Gln Ser Val
        115                 120                 125

Asn Tyr Leu Met Gly Thr Trp Pro Phe Gly Thr Ile Leu Cys Lys Ile
    130                 135                 140

Val Ile Ser Ile Asp Tyr Tyr Asn Met Phe Thr Ser Ile Phe Thr Leu
145                 150                 155                 160

Cys Thr Met Ser Val Asp Arg Tyr Ile Ala Val Cys His Pro Val Lys
                165                 170                 175

Ala Leu Asp Phe Arg Thr Pro Arg Asn Ala Lys Ile Ile Asn Val Cys
            180                 185                 190

Asn Trp Ile Leu Ser Ser Ala Ile Gly Leu Pro Val Met Phe Met Ala
        195                 200                 205

Thr Thr Lys Tyr Arg Gln Gly Ser Ile Asp Cys Thr Leu Thr Phe Ser
    210                 215                 220

His Pro Thr Trp Tyr Trp Glu Asn Leu Leu Lys Ile Cys Val Phe Ile
225                 230                 235                 240

Phe Ala Phe Ile Met Pro Val Leu Ile Ile Thr Val Cys Tyr Gly Leu
                245                 250                 255

Met Ile Leu Arg Leu Lys Ser Val Arg Met Leu Ser Gly Ser Lys Glu
            260                 265                 270

Lys Asp Arg Asn Leu Arg Arg Ile Thr Arg Met Val Leu Val Val Val
        275                 280                 285

Ala Val Phe Ile Val Cys Trp Thr Pro Ile His Ile Tyr Val Ile Ile
    290                 295                 300

Lys Ala Leu Val Thr Ile Pro Glu Thr Thr Phe Gln Thr Val Ser Trp
305                 310                 315                 320

His Phe Cys Ile Ala Leu Gly Tyr Thr Asn Ser Cys Leu Asn Pro Val
                325                 330                 335

Leu Tyr Ala Phe Leu Asp Glu Asn Phe Lys Arg Cys Phe Arg Glu Phe
            340                 345                 350

Cys Ile Pro Thr Ser Ser Asn Ile Glu Gln Gln Asn Ser Thr Arg Ile
        355                 360                 365

Arg Gln Asn Thr Arg Asp His Pro Ser Thr Ala Asn Thr Val Asp Arg
    370                 375                 380

Thr Asn His Gln Leu Glu Asn Leu Glu Ala Glu Thr Ala Pro Leu Pro
385                 390                 395                 400

## Beschreibung

Die vorliegende Erfindung betrifft ein Verfahren zur Bestimmung von Analyten, insbesondere illegalen Drogen, in einer Probe, sowie Testelemente, Probennahmeelemente und Kits, welche zur Durchführung des Verfahrens geeignet sind.

Immunoassays, welche beispielsweise auf der "Lateral Flow Technologie" basieren, sind weltweit verbreitete und anerkannte Schnelltestsysteme zum Nachweis von Analyten in Körperflüssigkeiten oder auf Oberflächen. Im Rahmen derartiger Tests wird eine den Analyten enthaltende Probe üblicherweise mit einem geeigneten Probennahmeelement von einer Oberfläche aufgenommen und auf das Testelement (z.B. auf einen Teststreifen) übertragen. Der Nachweis im Testelement erfolgt dann mittels einer immunologischen Nachweisreaktion, welche auf der Bildung von Immunkomplexen aus Antigenen und Antikörpern basieren.

Immunologische Nachweissysteme bieten aufgrund der biophysikalischen und biochemischen Eigenschaften der Antigen-Antikörper-Bindung im Allgemeinen eine hohe Spezifität und Sensitivität. Dies ist speziell beim Nachweis von illegalen Drogen (z.B. Amphetaminen, Metamphetaminen, Cannabis, Kokain, Heroin) von herausragender Bedeutung. Hierbei besteht zum einen das Bedürfnis, den Konsum illegaler Drogen schnell nachzuweisen. Andererseits sollten die Testformate auch eine hohe Sensitivität und Spezifität besitzen, um falsch-positive wie falsch-negative Testergebnisse auszuschließen und eine zuverlässige Aussage darüber zu liefern, welche Droge konsumiert wurde.

Seit dem Jahr 2008 sind Mischungen verschiedener Kräuter und Aromastoffe erhältlich, welche laut Verpackungshinweis zum Verräuchern und "Beduften" von Räumen angewendet werden sollen. Fakt ist jedoch, dass diese Kräutermischungen direkt oder in Kombination mit Tabak durch Inhalation des entstehenden Rauches in gleicher Weise wie Cannabis als Rauschdroge konsumiert werden. Durch professionelle Vermarktung, starke Präsenz in den Massenmedien und die allgemeine, aber irrige Annahme, es handle sich um als "Legal Highs" bezeichnete legal erhältliche Kräutermischungen mit "Cannabis-ähnlicher" Rauschwirkung, haben beispielsweise "Spice", "Smoke", "Lava Red" und eine Vielzahl an Folgeprodukten eine große Popularität erlangt.

Chemische Analysen zeigen indessen, dass die pharmakologische Wirkung derartiger Kräutermischungen auf synthetische Cannabinoide, welche auf den Kräuterblättern imprägniert sind, zurückzuführen ist und die Kräuterblätter lediglich als Trägermaterial dienen. So ist beispielsweise seit dem Jahr 2008 bekannt, dass nicht-deklarierte synthetische Cannabinoide wie JWH-018 und CP-47497 für die Wirkung von "Spice" verantwortlich sind. Bei den synthetischen Cannabinoiden handelt sich um eine Gruppe von mehr als 500 Substanzen, von denen ein Großteil in den 1980er und 1990er Jahren als mögliche Rezeptoragonisten für die humanen Cannabinoidrezeptoren hergestellt wurden. Ziel war die Entwicklung neuer analgetisch wirksamer Arzneistoffe ohne die psychoaktive Komponente von Cannabis.

Neben bestehendem Suchtpotential ist der Konsum synthetischer Cannabinoide (vor allem bei Langzeitkonsum) mit einem hohen Risiko einer Gesundheitsgefährdung verbunden, da keine verlässlichen Daten zu Dosis-Wirkungsbeziehungen, unerwünschten Nebenwirkungen oder Toxikologie existieren. Konsumenten von Produkten, welche synthetische Cannabinoide enthielten, berichten von Cannabis-ähnlicher Wirkung inklusive der für Cannabiskonsum üblichen unerwünschten Nebeneffekte, wie etwa Schwindelgefühl, erhöhter Ängstlichkeit, Herzrasen und Benommenheit (Vandrey et al., Drug Alcohol Depend. (2012), 120, 238-241).

Neben den weitestgehend unbekannten Gesundheitsrisiken für die Konsumenten bestehen auch Risiken für die Allgemeinheit. Dies ist beispielsweise dann der Fall, wenn Personen am Arbeitsplatz oder bei der Teilnahme am Straßenverkehr unter dem Einfluss synthetischer Cannabinoide stehen, wodurch vergleichbare Sicherheitsrisiken wie beim Konsum anderer illegaler Drogen, wie beispielsweise von Amphetaminen, Benzodiazepinen, Ketaminen, Kokain, Methamphetaminen, Cannabis und Opioiden, entstehen. Dies führte zum strikten Verbot dieser Substanzen bzw. diese Substanzen enthaltender Produkte in einer Vielzahl von Ländern, u.a. in großen Teilen Europas.

Ungeachtet dieses Verbots werden Produkte, welche synthetische Cannabinoide beinhalten, vor allem über das Internet weiterhin verkauft. Dementsprechend ist es insbesondere bei Straßenverkehrskontrollen von Polizei und Zoll, beim Nachweis von Drogenschmuggel oder bei Arbeitsplatzkontrollen entscheidend, sensitive und zuverlässige Drogenschnelltests zum Nachweis synthetischer Cannabinoide zur Verfügung zu haben. Allerdings können synthetische Cannabinoide mit den derzeit verfügbaren Drogenschnelltests nicht oder nur unzureichend nachgewiesen werden, da gängige Drogenschnelltests diese Substanzklasse nicht oder nur teilweise erfassen.

EP 0 699 906 A2 offenbart einen Drogenschnelltest, welcher als Oberflächen-, Schweiß- bzw. Speicheltest unter der Bezeichnung DrugWipe^{®} (Firma Securetec Detektions-Systeme AG) kommerziell erhältlich ist. Der Test bedient sich eines im Wesentlichen aus Kunststoff bestehenden Wischelements ("Wischer") mit aufgeschweißtem Vlies, mittels welchem eine Probe des Analyten (z.B. von einer Oberfläche oder aus einer Lösung) genommen und anschließend direkt auf einen immunchromatographischen Teststreifen ("Lateral Flow"-Technologie), welcher in einem Einweggehäuse gelagert ist, übertragen wird.

Durch Inkontaktbringen des Teststreifens mit einer wässrigen Lösung (Wasser oder Puffer mit verschiedenen Reagenzien) wird die Chromatographie gestartet, wobei das Ergebnis der Bestimmung mit dem Auge oder unter Verwendung einer geeigneten Messvorrichtung ausgelesen werden kann. Der Nachweis des Analyten erfolgt dadurch, dass an Drogenmoleküle (Antigene) gebundene Antikörper an einer Testlinie abbinden und aufgrund ihrer Goldmarkierung eine farbige Linie bilden, welche im Auslesefenster optisch detektiert werden kann. Antikörper, welche nicht mit Drogenmolekülen beladen sind, werden vor Erreichen der Testlinie mittels Polyhaptenen, die auf dem Teststreifen in immobilisierter Form vorliegen, abgefangen und sind optisch im Auslesefenster nicht sichtbar.

Im Vergleich zu allen anderen auf dem Markt befindlichen Drogenschnelltests ist der DrugWipe^{®} das einzige Produkt, welches bei Vorhandensein eines bestimmten Analyten in einer Probe eine farbige Linie ausbildet und insofern eine sogenannte Positivanzeige aufweist. Alle übrigen kommerziellen Produkte verfügen über eine Negativanzeige, bei der das Nicht-Erscheinen einer Linie als positiver Nachweis für den jeweiligen Analyten gewertet wird. Ein weiterer Vorteil des DrugWipe^{®} ist das geringe Probenvolumen, welches zum Nachweis von Drogen und anderen Substanzen benötigt wird. Während im Falle von DrugWipe^{®} ein Probenvolumen von kleiner 25 µl ausreichend ist, erfordern andere kommerzielle Tests ein Probenvolumen von mindestens 100 µl bis hin zu mehreren Millilitern.

Das geringe Probenvolumen, welches zur Durchführung des DrugWipe^{®}-Drogenschnelltests benötigt wird, stellt einen entscheidenden Vorteil gegenüber anderen kommerziell verfügbaren Testsystemen dar, da Drogenkonsumenten aufgrund der physiologischen Wirkung der Drogen häufig einen sehr trockenen Mund haben und nur wenig Speichel für die Probenahme zur Verfügung steht. Dies ist vor allem bei Konsumenten von Cannabis und synthetischen Cannabinoiden von Vorteil, da der Konsum dieser Drogen immer mit einem sehr trockenen, speichelarmen Mundraum einhergeht. Folglich stehen - gerade in solchen Fällen - oft nur sehr geringe Probenvolumina zur Verfügung, welche mit Ausnahme des DrugWipe^{®}-Drogenschnelltests allgemein nicht ausreichen, um ein vertrauenswürdiges bzw. fehlerfreies Ergebnis zu erhalten. Sofern die Durchführung eines Drogenschnelltests überhaupt möglich ist, dauert die Probennahme häufig mehrere Minuten, was weder dem Probanden noch dem Probenehmenden zumutbar ist.

Der von der Firma Varian entwickelte Schnelltest OraLab^{®} 6 dient zum Nachweis von Drogen aus Speichelproben und basiert ebenfalls auf der Lateral-Flow-Technologie. Während die Spezifität des Tests bei etwa 90-100% liegt, beträgt die Sensitivität bei Amphetaminen und Opiaten lediglich zwischen 50 und 90%, bei Δ⁹-Tetrahydrocannabinol und Kokain zum Teil sogar deutlich unter 50% (siehe DRUID-Studie, veröffentlicht auf der TIAFT 2009 in Genf). Ein großer Nachteil dieses Tests liegt weiterhin darin, dass relativ große Probenvolumina benötigt werden, welche bei akuten Drogenkonsumenten oft nicht zur Verfügung stehen.

Der Drogenschnelltest DrugTest^{®} 5000 der Firma Dräger, welcher ebenfalls auf der Lateral-Flow-Technologie basiert, stellt gemäß den Daten der DRUID-Studie (TIAFT 2009, Genf) ein zuverlässiges Testsystem zum Nachweis von Drogen dar. Allerdings besitzt dieses Testformat zwei signifikante Nachteile. Zum einen können die Ergebnisse auf dem Teststreifen ausschließlich mittels eines Auslesegeräts ausgelesen werden, welches in der Anschaffung sehr teuer und für den harten Feldeinsatz im Freien nur teilweise geeignet ist. Zum anderen werden für diesen Test 300 µl Speichel als Probenvolumen benötigt, weshalb die Probenahme, vor allem bei Drogenkonsumenten mit trockenem Mund, teilweise mehrere Minuten dauert. Insofern ergeben sich für diesen Test erhebliche Probleme in Bezug auf Wirtschaftlichkeit und einfache Handhabbarkeit.

Der von der Firma Mavand angebotene Testkit Rapid STAT^{®} stellt einen weiteren kommerziell erhältlichen Drogenschnelltest dar, bei welchem eine mit Puffer verdünnte Speichelprobe auf einem immunchromatographischen Teststreifen mit markierten Bindungspartnern inkubiert wird und welcher laut Herstellerangaben einen Nachweis bis zu einer unteren Grenze von 15 ng/ml an Δ⁹-Tetrahydrocannabinol ermöglicht. Ein signifikanter Nachteil dieses Tests ist dessen äußerst komplexe und umständliche Handhabung, welche ihn gerade für eine Anwendung bei der Verkehrspolizei ungeeignet macht, sowie die vergleichsweise geringe Spezifität für Δ⁹-Tetrahydrocannabinol von 80-90% (siehe DRUID-Studie, TIAFT 2009, Genf). Laut einer Studie der Universität Mainz liegt die Rate von falschpositiven Testergebnissen für Δ⁹-Tetrahydrocannabinol bei mehr als 10% und die Gesamtspezifität bei 84% (veröffentlicht auf der GTFCH 2009, Mosbach, http://www.gtfch.org/cms/images stories/media/tk/tk76_2/ abstractsposter.pdf).

WO 2005/075982 A2, EP 0 811 842 A2 und EP 0 699 906 A2 offenbaren Verfahren zur Bestimmung von Analyten in einer Körperflüssigkeit bzw. auf einer kontaminierten Oberfläche. Zur Durchführung des Verfahrens wird hierbei jeweils ein Testkit verwendet, welcher einen Teststreifen aus einem oder mehreren kapillaraktiven chromatografiefähigen Materialien, ein Probennahmeelement separat zur Teststreifenoberfläche, sowie eine Andrückvorrichtung zum Inkontaktbringen von Teststreifenoberfläche und Probennahmeelement umfasst. Hierbei wird zumindest ein Teil der Probe von dem Probennahmeelement auf das Testelement übertragen und der Analyt, nach dem Start der Chromatographie und Bindung an einen analytspezifischen Bindungspartner, analysiert.

WO 2005/121793 A2 offenbart ein Verfahren zum Nachweis eines Methamphetamins in einer flüssigen Probe. Zur Durchführung des Verfahrens wird vorzugsweise ein Kit verwendet, welcher einen chromatografischen Teststreifen und gegebenenfalls ein Probennahmeelement umfasst. Der Teststreifen umfasst seinerseits (a) ein trockenes poröses Material, auf welchem ein Pseudoephedrin/Träger-Konjugat oder ein Antikörper, der sowohl an das Methamphetamin als auch an das Konjugat binden kann, in einer Detektionszone immobilisiert ist, und (b) eine hiervon getrennte Markerfreisetzungszone, welche entweder den Antikörper in markierter Form, sofern die Detektionszone immobilisiertes Pseudoephedrin/Träger-Konjugat enthält, oder detektierbares Pseudoephedrin/Träger-Konjugat, sofern die Detektionszone immobilisierten Antikörper enthält, in die Flüssigkeit freisetzen kann.

Ein Nachteil der in EP 0 699 906 A2, EP 0 811 842 A2, WO 2005/075982 A2 und WO 2005/121793 A2 beschriebenen Nachweisverfahren besteht weiterhin darin, dass vor dem Beginn der Chromatographie keine Vorbehandlung der Probe vorgenommen wird und der analytspezifische Bindungspartner nicht direkt mit dem Analyten inkubiert wird. Vielmehr kommen der Analyt und der analytspezifische Bindungspartner erst im Laufe der Chromatographie miteinander in Kontakt, wodurch vor allem die Sensitivität des Verfahrens negativ beeinflusst wird und zumindest teilweise die gesetzlich geforderten Mindestnachweisgrenzen für Drogenmoleküle nicht erreicht werden.

Darüber hinaus besitzen die in WO 2005/075982 A2, EP 0 811 842 A2, EP 0 699 906 A2 und WO 2005/121793 A2 beschriebenen Verfahren den Nachteil, dass es sich bei dem analytspezifischen Bindungspartner jeweils um einen Antikörper handelt. Dies ist insofern problematisch, als der Nachweis unterschiedlicher Drogenmoleküle, wie beispielsweise der Nachweis von Δ⁹-Tetrahydrocannabinol und verschiedensten synthetischen Cannabinoiden, üblicherweise nicht mittels eines einzigen Antikörpers erfolgen kann, da Antikörper in der Regel eine eingeschränkte Selektivität besitzen und keinen breitbandigen Nachweis von Substanzen mit unterschiedlicher chemischer Struktur erlauben. Sofern die gleichzeitige Bestimmung von Δ⁹-Tetrahydrocannabinol und verschiedensten synthetischen Cannabinoiden in einem einzigen Test erfolgen soll, müssten daher unter Umständen mehrere hundert Antikörper entwickelt werden, was weder technisch noch wirtschaftlich sinnvoll ist.

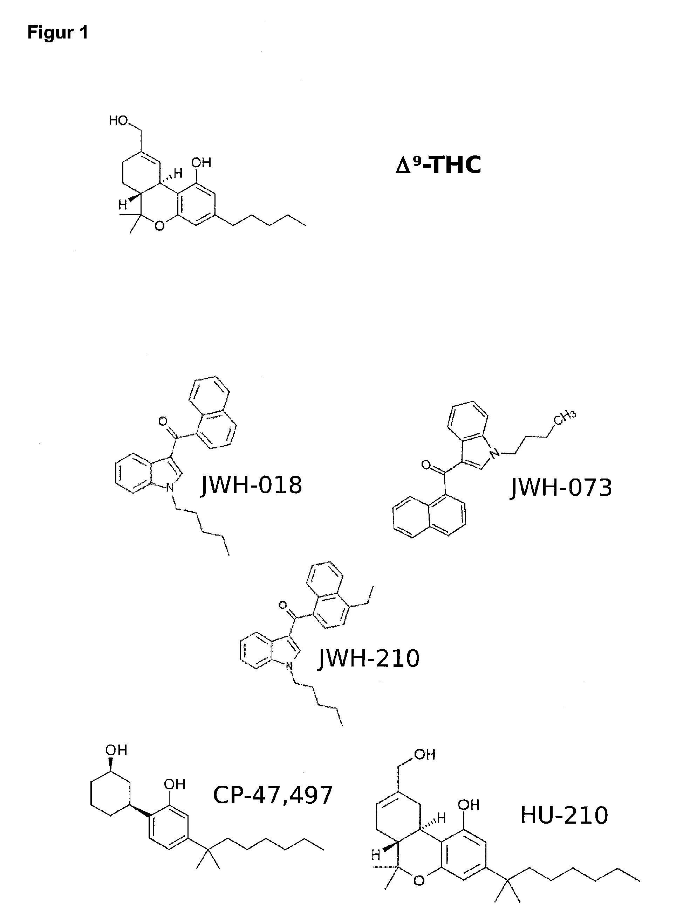

Der Grund, weshalb herkömmliche, Antikörper-basierte Drogenschnelltests im Allgemeinen keine Bestimmung synthetischer Cannabinoide ermöglichen, ist, dass die synthetischen Cannabinoide eine Vielzahl von Substanzen umfassen, welche ihrerseits eine hohe strukturelle Variabilität zeigen (d.h. untereinander nur geringfügige oder keinerlei strukturelle Ähnlichkeiten aufweisen). Ferner besteht üblicherweise auch keine ausgeprägte strukturelle Ähnlichkeit zum natürlichen Cannabiswirkstoff Δ⁹-Tetrahydrocannabinol, wie beispielsweise aus Figur 1 hervorgeht. Dementsprechend erfordert der Nachweis synthetischer Cannabinoide üblicherweise eine aufwändige, Geräte-basierte Laboruntersuchung, welche weder zeit- noch ortsnah durchführbar ist.

Der einzige derzeit verfügbare Schnelltest für synthetische Cannabinoide wird von der Firma DrugCheck (http://www.drugcheck.com/dc_info-k2-spice.html) angeboten, wobei allerdings lediglich die Substanzen JWH-018 und JWH-073 (siehe Figur 1) nachgewiesen werden können. Darüber hinaus handelt es sich bei diesem Schnelltest um einen Urintest, welcher aufgrund der komplexen Probennahme in der Praxis ungern angewendet wird und zudem lediglich Metaboliten der zu bestimmenden Substanzen nachweist (und nicht die tatsächlichen psychoaktiven Substanzen). Zudem können die Metaboliten zum Teil noch mehrere Wochen nach dem tatsächlichen Konsum detektiert werden, obwohl zu diesem Zeitpunkt keinerlei psychoaktive Beeinträchtigung des Konsumenten mehr vorliegt.

Unter Berücksichtigung dieser Sachverhalte bestand die der vorliegenden Erfindung zugrunde liegende Aufgabe somit darin, ein Verfahren zur Bestimmung von Analyten, insbesondere zur Bestimmung von illegalen Drogen, bereitzustellen, bei welchem die Nachteile des Standes der Technik zumindest teilweise beseitigt sind. Insbesondere sollte das Verfahren eine einfache, schnelle und zuverlässige Vor-Ort-Bestimmung einer Vielzahl von Cannabinoiden und Opioiden, speziell von Δ⁹-Tetrahydrocannabinol und synthetischen Cannabinoiden, ermöglichen, welche mit einem kleinen Probenvolumen durchgeführt werden kann und gleichzeitig eine hohe Sensitivität und Spezifität gewährleistet.

Diese Aufgabe wird erfindungsgemäß gelöst durch ein Verfahren zur Bestimmung eines Analyten in einer Probe, umfassend die Schritte:
(a) Bereitstellen eines Testelements,
(b) Aufbringen der Probe auf das Testelement und
(c) Bestimmen des Vorhandenseins oder/und der Menge des Analyten auf dem Testelement,
wobei das Testelement mindestens ein den Analyten bindendes Rezeptormolekül umfasst, wobei die ligandenbindende Domäne des Rezeptormoleküls in nativer, verkürzter oder mutierter Form vorliegt und gegebenenfalls mit einem heterologen Molekül konjugiert ist.

Der erste Schritt des erfindungsgemäßen Verfahrens erfordert die Bereitstellung eines für die Bestimmung des Analyten geeigneten Testelements, welches mindestens ein den Analyten bindendes Rezeptormolekül umfasst. Die zu diesem Zweck verwendeten Testelemente umfassen grundsätzlich jede dem Fachmann geläufige physikalische Form, welche für die Bestimmung des Vorhandenseins oder/und der Menge eines Analyten in einer Probe geeignet ist. Hierbei ist das Testelement vorzugsweise derart ausgebildet, dass es bei Anwesenheit des zu bestimmenden Analyten ein optisch detektierbares Signal erzeugt, welches eine qualitative oder/und quantitative Bestimmung des Analyten ermöglicht.

Beispiele für Testelemente im Sinne der vorliegenden Erfindung umfassen insbesondere Testelemente zur Durchführung eines heterogenen, homogenen oder chromatographischen Tests, eines ELISA (Enzyme-linked Immunosorbent Assay) oder eines FRET-Assays (Fluorescence Resonance Energy Transfer). Derartige Testelemente sind im Fachbereich bekannt und können vom Fachmann entsprechend den jeweiligen Anforderungen gezielt ausgewählt werden. Sofern gewünscht bzw. erforderlich, können die Testelemente mikrofluidische Strukturen, wie beispielsweise Mikrokanäle, Stufen, Verzweigungen oder/und Kammern, enthalten, wodurch gegebenenfalls ein verbesserter Transfer der Probe in dem Testelement erzielt werden kann. In einer bevorzugten Variante der Erfindung handelt es sich bei dem Testelement um einen chromatographischen Teststreifen, auf den der Analyt beispielsweise in Form einer wässrigen oder nichtwässrigen Lösung aufgebracht werden kann.

In einer Variante der Erfindung ist der chromatographische Teststreifen aus einem einzigen chromatographiefähigen, gegebenenfalls streifenförmigen Material gebildet. Vorzugsweise umfasst der chromatographische Teststreifen allerdings mehrere, auf einer Trägerschicht überlappend angeordnete kapillaraktive Flächen aus gleichen oder unterschiedlichen chromatographischen Materialien, welche in fluider Kommunikation miteinander stehen und auf diese Weise eine Transportstrecke bilden, entlang derer ein Fluid, durch Kapillarkräfte getrieben, durch sämtliche Bereiche des Testelements strömen kann. Als chromatographisches Material kann dabei jedes bekannte flüssigkeitsabsorbierende, poröse oder kapillaraktive Material verwendet werden, wie z.B. Cellulose und Derivate hiervon, Glasfasern, sowie Vliese und Gewebe aus künstlichen oder natürlichen Materialien. Chromatographische Teststreifen, welche im Rahmen der vorliegenden Erfindung Anwendung finden können, sind beispielsweise in EP 0 699 906 A2 beschrieben, auf deren Offenbarung hiermit ausdrücklich Bezug genommen wird.

Das analytbindende Rezeptormolekül, welches in löslicher Form oder in immobilisierter Form auf dem Testelement vorliegen kann, umfasst definitionsgemäß eine ligandenbindende Domäne, welche gegebenenfalls mit einem heterologen Molekül konjugiert ist. Der Ausdruck "ligandenbindende Domäne", wie er in vorliegender Anmeldung verwendet wird, bezeichnet eine Sequenz von mehreren, d.h. mindestens zwei aufeinanderfolgenden Aminosäuren eines Rezeptormoleküls, über die ein zu bestimmender Analyt an das Rezeptormolekül bindet. Dementsprechend wird als analytbindendes Rezeptormolekül insbesondere ein Polypeptid oder Protein eingesetzt, welches sich bevorzugt von einem Membranprotein, stärker bevorzugt von einem Transmembranprotein, am stärksten bevorzugt von einem G-Protein-gekoppelten Rezeptor, ableitet. Sofern die ligandenbindende Domäne mit einem heterologen Molekül konjugiert ist, so handelt es sich bei dem heterologen Molekül bevorzugt um ein heterologes Polypeptid, stärker bevorzugt um eine Immunglobulindomäne.

Bevorzugt umfasst das analytbindende Rezeptormolekül die ligandenbindende Domäne, insbesondere die extrazelluläre ligandenbindende Domäne eines Betäubungsmittel-bindenden Rezeptormoleküls, wie beispielsweise eines Cannabinoid- oder Opioid-bindenden Rezeptormoleküls. Stärker bevorzugt gelangt im Rahmen der vorliegenden Erfindung ein Rezeptormolekül zum Einsatz, welches die ligandenbindende Domäne, insbesondere die extrazelluläre ligandenbindende Domäne eines Rezeptormoleküls ausgewählt aus der Gruppe bestehend aus Cannabinoidrezeptor 1, Cannabinoidrezeptor2, Opioidrezeptor δ, Opioidrezeptor κ, Opioidrezeptor u1 und Opioidrezeptor µ2 umfasst.

Erfindungsgemäß kann die ligandenbindende Domäne eine native ligandenbindende Domäne, eine verkürzte Form einer nativen ligandenbindenden Domäne oder eine mutierte Form einer nativen ligandenbindenden Domäne sein, wobei der Ausdruck "native ligandenbindende Domäne" die ligandenbindende Domäne eines natürlich vorkommenden Rezeptormoleküls bezeichnet und das natürlich vorkommende Rezeptormolekül vorzugsweise aus einem Säuger, insbesondere aus einem Menschen, stammt. Natürlich vorkommende Rezeptormoleküle, welche im Rahmen des erfindungsgemäßen Verfahrens besonders bevorzugt Anwendung finden, umfassen den humanen Cannabinoidrezeptor 1 (siehe Figuren 6 und 7), den humanen Cannabinoidrezeptor 2 (siehe Figuren 8 und 9), den humanen Opioidrezeptor κ (siehe Figuren 10 und 11), den humanen Opioidrezeptor µ1 und den humanen Opioidrezeptor µ2.

Bevorzugt kann im Rahmen des hierin beschriebenen Verfahrens ein analytbindendes Rezeptormolekül zum Einsatz gelangen, welches eine gegenüber einem natürlich vorkommenden Rezeptormolekül verkürzte Aminosäuresequenz aufweist oder/und welches eine verkürzte Form einer nativen ligandenbindenden Domäne umfasst. Der Ausdruck "verkürzte Form einer nativen ligandenbindenden Domäne", wie er in vorliegender Anmeldung verwendet wird, bezeichnet jegliches Fragment einer nativen ligandenbindenden Domäne, welches zur Bindung des Analyten befähigt ist und eine gegenüber der ligandenbindenden Domäne eines natürlich vorkommenden Rezeptormoleküls verkürzte Aminosäuresequenz, beispielsweise eine zwischen 5% und 75% verkürzte Aminosäuresequenz, aufweist, wobei die Verkürzung der Aminosäuresequenz am N-Terminus, am C-Terminus oder/und zwischen dem N-Terminus und dem C-Terminus des natürlich vorkommenden Rezeptormoleküls bzw. der nativen ligandenbindenden Domäne des Rezepormoleküls realisiert sein kann.

Sofern das analytbindende Rezeptormolekül eine verkürzte Aminosäuresequenz aufweist, so ist es erfindungsgemäß bevorzugt, dass die Aminosäuresequenz um bis zu 10%, 20%, 30%, 40% oder 50% gegenüber der Aminosäuresequenz des nativen analytbindenden Rezeptormoleküls verkürzt ist. Als besonders bevorzugt hat sich indessen der Einsatz eines analytbindenden Rezeptormoleküls erwiesen, in welchem die Aminosäuresequenz der ligandenbindenden Domäne um bis zu 10%, 20%, 30%, 40% oder 50% gegenüber der Aminosäuresequenz der ligandenbindenden Domäne eines natürlich vorkommenden Rezeptormoleküls, wie beispielsweise der Aminosäuresequenz der ligandenbindenden Domäne von humanem Cannabinoidrezeptor 1, humanem Cannabinoidrezeptor 2, humanem Opioidrezeptor κ, humanem Opioidrezeptor µ1 oder humanem Opioidrezeptor µ2, verkürzt ist.

Weiterhin kann in dem hierin beschriebenen Verfahren insbesondere ein analytbindendes Rezeptormolekül zum Einsatz gelangen, welches eine gegenüber einem natürlich vorkommenden Rezeptormolekül mutierte Aminosäuresequenz aufweist oder/und welches eine mutierte Form einer nativen ligandenbindenden Domäne umfasst. Der Ausdruck "mutierte Form einer nativen ligandenbindenden Domäne", wie er in vorliegender Anmeldung verwendet wird, bezeichnet eine genetisch veränderte Variante einer nativen ligandenbindenden Domäne, welche zur Bindung des Analyten befähigt ist und eine gegenüber der ligandenbindenden Domäne eines natürlich vorkommenden Rezeptormoleküls veränderte Aminosäuresequenz, beispielsweise eine Aminosäuresequenz mit einer Sequenzidentität zwischen 50% und 99%, aufweist, wobei die Veränderung der Aminosäuresequenz am N-Terminus, am C-Terminus oder/und zwischen dem N-Terminus und dem C-Terminus des natürlich vorkommenden Rezeptormoleküls bzw. der nativen ligandenbindenden Domäne des Rezepormoleküls realisiert sein kann.

Die Mutationen können natürlichen Ursprungs sein (z.B. Punktmutationen bzw. Transkript- oder Splicevarianten eines natürlich vorkommenden Gens) oder unter Verwendung von im Fachbereich bekannten rekombinanten Methoden eingeführt werden, wie beispielsweise durch ortsspezifisches Entfernen, Austauschen oder/und Hinzufügen von Nukleotiden auf DNA-Ebene bzw. von Aminosäuren auf Protein-Ebene. Auf diese Weise resultiert mindestens ein Aminosäureaustausch innerhalb der Aminosäuresequenz des natürlich vorkommenden Rezeptormoleküls, insbesondere innerhalb der Aminosäuresequenz der nativen ligandenbindenden Domäne, wodurch beispielsweise eine Erhöhung der thermischen oder/und chemischen Stabilität des Rezeptormoleküls erzielt werden kann. Ziel ist es, die Bindeeigenschaften des nativen Rezeptormoleküls gegenüber dem Analyten aufrecht zu erhalten und gleichzeitig dessen Stabilität für eine Anwendung in einem Testelement zu erhöhen, wobei speziell eine Stabilisierung in Zusammenhang mit den Prozessen des Eintrocknens (Dehydrierung), der Lagerung über 1-2 Jahre im trockenen Zustand und des Rehydrierens von Interesse sind.

Weist das analytbindende Rezeptormolekül eine mutierte Aminosäuresequenz auf, so ist es bevorzugt, dass diese Aminosäuresequenz zu mindestens 80%, insbesondere zu mindestens 95% identisch ist mit der Aminosäuresequenz des nativen analytbindenden Rezeptormoleküls. Besonders bevorzugt ist in diesem Zusammenhang die Verwendung eines analytbindenden Rezeptormoleküls, in welchem die Aminosäuresequenz der ligandenbindenden Domäne zu mindestens 80%, insbesondere zu mindestens 95% identisch ist mit der Aminosäuresequenz der ligandenbindenden Domäne eines natürlich vorkommenden Rezeptormoleküls, wie beispielsweise der Aminosäuresequenz der ligandenbindenden Domäne von humanem Cannabinoidrezeptor 1, humanem Cannabinoidrezeptor 2, humanem Opioidrezeptor κ, humanem Opioidrezeptor µ1 oder humanem Opioidrezeptor µ2.

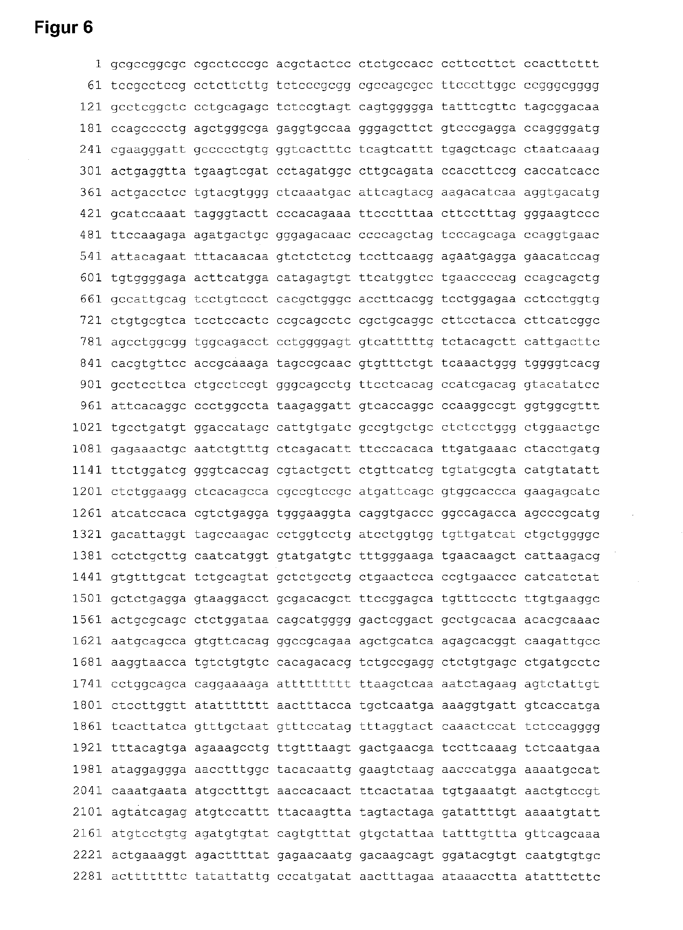

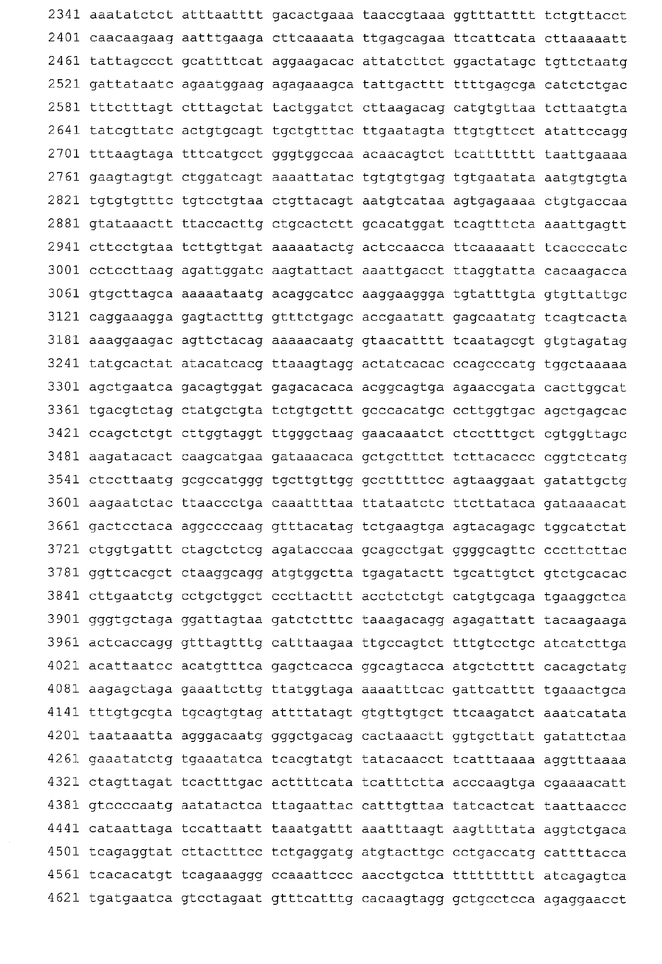

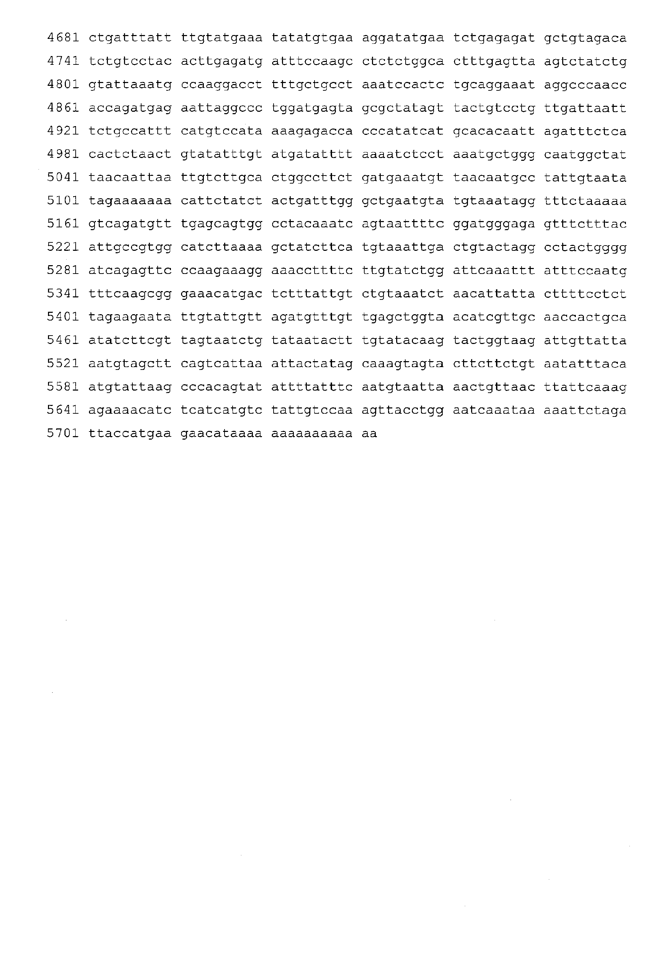

Konkrete Beispiele für Rezeptormoleküle bzw. ligandenbindende Domänen, welche im Rahmen des erfindungsgemäßen Verfahrens Anwendung finden können, umfassen neben den in den Figuren 6-13 der vorliegenden Anmeldung dargestellten Cannabinoid- und Opioidrezeptoren u.a. die in WO 92/02640 A1, WO 95/07983 A1, WO 98/33937 A2, WO 00/04046 A2, WO 03/002718 A2, WO 2004/007551 A1, US 6,235,496 B1 und US 2002/0077285 A1 beschriebenen natürlichen oder rekombinant hergestellten DNA- und Aminosäuresequenzen. Auf die Offenbarung vorstehend genannter Dokumente wird hiermit ausdrücklich Bezug genommen.

Ein wesentlicher Vorteil des erfindungsgemäßen Verfahrens ist es, dass die Analyten mit hoher Spezifität und Sensitivität nachgewiesen werden können. Insbesondere jedoch kann mit Hilfe eines einzigen Rezeptormoleküls der spezifische und sensitive Nachweis strukturell verschiedenster Analyten aus einer bestimmten Substanzklasse geführt werden. So ist es beispielsweise möglich, unter Verwendung der ligandenbindenden Domänen von humanem Cannabinoidrezeptor 1 und humanem Cannabinoidrezeptor 2 sowohl Δ⁹-Tetrahydrocannabinol als auch alle derzeit bekannten sowie künftigen synthetischen Cannabinoide zu erfassen.

Grund hierfür ist, dass eine Substanz dann als illegales Cannabinoid eingestuft wird, wenn sie im Menschen eine zu Δ⁹-Tetrahydrocannabinol vergleichbare psychoaktive Wirkung entfaltet. Dies kann allerdings nur dann der Fall sein, wenn die Substanz nach Konsum durch die Bindung an einen humanen oder beide humane Cannabinoidrezeptoren die für Cannabinoide bekannten Signaltransduktionswege auslöst. Gleichermaßen ist es demzufolge möglich, mittels der ligandenbindenden Domänen von humanen Opioidrezeptoren (z.B. von Opiodrezeptor Δl bzw. Opioidrezeptor µ2) strukturell unterschiedliche Moleküle aus der Substanzklasse der Opioide, wie beispielsweise Codein, Desomorphin, Fentanyl, Heroin, Methadon und Morphin, zu erfassen.

Um den Nachweis des Analyten mittels der hierin beschriebenen Testelemente zu erleichtern, kann das analytbindende Rezeptormolekül eine detektierbare Markierung, wie beispielsweise eine Enzymmarkierung, Farbstoffmarkierung, Fluoreszenzmarkierung oder Partikelmarkierung, umfassen. Im Rahmen der vorliegenden Erfindung hat sich eine Partikelmarkierung, bei der das analytbindende Rezeptormolekül kovalent oder nicht-kovalent an die Oberfläche geeigneter Nanopartikel (z.B. Gold - oder Platinpartikel) gebunden ist, als vorteilhaft erwiesen. Besonders bevorzugt handelt es sich bei der Markierung um eine Goldmarkierung, welche den Vorteil besitzt, dass das Testergebnis vom Anwender direkt optisch-visuell erfasst und ausgewertet werden kann. Techniken, mittels welcher oben beschriebene Markierungen in ein zu markierendes Molekül eingeführt werden können, sind dem Fachmann bekannt und werden insofern nicht näher erläutert.

Zur Bestimmung des Analyten unter Verwendung der hierin beschriebenen Testelemente wird eine den Analyten enthaltende Probe in einem weiteren Schritt des erfindungsgemäßen Verfahrens unter Verwendung geeigneter Mittel auf das Testelement aufgebracht. In einer bevorzugten Variante wird die Probe hierbei mittels eines Probennahmeelements, welches eine oder mehrere Probennahmeflächen aufweisen kann, von einer Oberfläche aufgenommen und das Probennahmeelement anschließend mit dem Testelement in Kontakt gebracht, wobei zumindest ein Teil der Probe von dem Probennahmeelement auf das Testelement übertragen wird. Die Probennahme kann hierbei grundsätzlich auf beliebige Weise erfolgen, wie beispielsweise durch Abkratzen, Abwischen oder Absaugen der Probe von einer geeigneten Oberfläche, insbesondere einer Körperoberfläche wie beispielsweise Zunge oder Haut.

Bevorzugt handelt es sich bei dem Probennahmeelement um ein Wischelement, welches das Abwischen einer Probe von einer zu untersuchenden Oberfläche sowie einen nachfolgenden Transfer der Probe auf das Testelement, beispielsweise unter Ausnutzung von Kapillareffekten, ermöglicht. Das Wischelement weist eine oder mehrere (voneinander unabhängige) Wischflächen auf, wobei im Falle mehrerer Wischflächen Wischelemente mit 2, 3 oder 4 Wischflächen bevorzugt sind. Sofern ein Wischelement mit mehreren Wischflächen zum Einsatz gelangt, kommen üblicherweise auch mehrere (voneinander unabhängige) Testelemente zum Einsatz, wobei jedes dieser Testelemente jeweils mit einer Wischfläche des Wischelements in Kontakt gebracht wird.

Die mindestens eine Wischfläche, welche vorzugsweise mit einer Oberfläche des Wischelements verschweißt ist, kann grundsätzlich aus einem beliebigen Material bestehen, das dem Fachmann für die Zwecke der vorliegenden Erfindung nützlich erscheint und eine spätere Übertragung des Analyten auf das Testelement nicht negativ beeinflusst. Als zweckmäßig haben sich hierbei speziell saugfähige Materialien, insbesondere Gewebe, Vliese oder/und poröse Matrices (z.B. Membranen und Schwämme), erwiesen. Besonders bevorzugt werden im Rahmen der Erfindung Vliese, insbesondere Vliese auf Basis von Cellulose-, Polyester- oder/und Glasfasern, eingesetzt, wobei die Fasern gegebenenfalls mit Hilfe eines organischen Bindemittels zusammengehalten werden können. Geeignete Vliese bzw. Fasern sind beispielsweise in DE 38 02 366 A1 und EP 0 699 906 A2 beschrieben, auf deren Offenbarung ausdrücklich Bezug genommen wird.

Was die äußere Ausgestaltung der Wischfläche(n) betrifft, so bestehen grundsätzlich keinerlei Einschränkungen hinsichtlich der Dicke, Dimension und Geometrie. Die Dicke der Wischfläche(n) bzw. des hierfür verwendeten Materials ist für die Zwecke der vorliegenden Erfindung von untergeordneter Bedeutung und liegt üblicherweise in einem Bereich von 0.1 bis 3 mm. Die Dimension der Wischfläche(n) ist vorteilhafterweise der Dimension des Testelements angepasst, d.h. die Breite der Wischfläche(n) sollte die Breite des Testelements weder übersteigen noch unterbieten. Bevorzugte Dimensionen der Wischfläche(n) liegen bezüglich der Länge im Bereich von 0.3 bis 2 cm, bezüglich der Breite im Bereich von 0.3 bis 1 cm.

Die Geometrie der Wischfläche(n) kann den jeweiligen Anforderungen an die zu untersuchende Oberfläche angepasst werden, wobei eine dreieckige, viereckige (z.B. quadratische, rechteckige, rautenförmige) oder rollenförmige Ausgestaltung der Wischfläche(n) als besonders vorteilhaft angesehen wird. Eine rollenförmige Ausgestaltung der Wischfläche(n) hat dabei den Vorteil, dass durch die große Oberfläche der Wischfläche(n) ein besonders guter Kontakt zwischen dem Wischelement und dem Testelement realisiert wird und dementsprechend eine Erhöhung der Sensitivität des Verfahrens erzielt werden kann.

Um eine besonders hohe Sensitivität und Spezifität bei der Bestimmung des Analyten zu gewährleisten, umfasst das Testelement oder/und das Probennahmeelement ein Analyt-Übertragungsreagenz. Das Analyt-Übertragungsreagenz, welches den Transfer des Analyten von der zu untersuchenden Oberfläche auf das Probennahmeelement oder/und die nachfolgende Übertragung des Analyten vom Probennahmeelement auf das Testelement insbesondere durch Blockierung freier Bindungsstellen auf dem Probennahmeelement oder/und Beeinflussung der Analyteigenschaften begünstigt, kann zu diesem Zweck beispielsweise auf dem Testelement oder/und dem Probennahmeelement, insbesondere im Bereich eventuell vorhandener Probennahmefläche(n), imprägniert sein.

In einer Variante der Erfindung umfasst das zur Bestimmung des Analyten eingesetzte Testelement oben beschriebenes Analyt-Übertragungsreagenz, während in einer anderen Ausführungsform das Probennahmeelement das Analyt-Übertragungsreagenz beinhaltet. Besonders bevorzugt umfassen indessen sowohl das Testelement als auch das Probennahmeelement ein Analyt-Übertragungsreagenz, welches jeweils mindestens eine analytunspezifische Substanz ausgewählt aus der Gruppe bestehend aus einem Protein, einem Proteingemisch, einem Kohlenhydrat und einem Zuckeralkohol enthält. Die Konzentration an analytunspezifischer Substanz in dem Analyt-Übertragungsreagenz kann entsprechend den jeweiligen Anforderungen an das Testelement vom Fachmann angepasst werden, beträgt jedoch üblicherweise etwa 0.01 bis etwa 15 Gew.-%, bezogen auf das Gesamtgewicht des Analyt-Übertragungsreagenzes.

Sofern das Testelement das Analyt-Übertragungsreagenz umfasst, so ist es ferner als bevorzugt anzusehen, dass das Analyt-Übertragungsreagenz ein analytunspezifisches Protein oder/und ein analytunspezifisches Proteingemisch, insbesondere ein analytunspezifisches Protein, enthält. Als Protein wird erfindungsgemäß bevorzugt eine Substanz ausgewählt aus der Gruppe bestehend aus Gelatine, Ovalbumin und Rinderserumalbumin eingesetzt, während als Proteingemisch beispielsweise Magermilchpulver zur Anwendung gelangen kann. Demgegenüber umfasst das Probennahmeelement vorzugsweise ein Analyt-Übertragungsreagenz, welches ein Kohlenhydrat oder/und einen Zuckeralkohol, insbesondere einen Zuckeralkohol, enthält.

Der Begriff "Kohlenhydrat", wie er in der vorliegenden Anmeldung verwendet wird, bezeichnet Monosaccharide und Oligosaccharide der allgemeinen Summenformel CₙH₂ₙOₙ, welche jeweils natürlichen oder synthetischen Ursprungs sein können. Als Monosaccharide gelangen insbesondere natürlich vorkommende Tetrosen, Pentosen und Hexosen, wie beispielsweise Erythrose, Threose, Ribose, Arabinose, Lyxose, Xylose, Allose, Altrose, Galactose, Glucose, Gulose, Idose, Mannose, Talose und Fructose, die jeweils in der D-Form oder in der L-Form vorliegen können, zum Einsatz. Als Oligosaccharide können insbesondere natürlich vorkommende Disaccharide und Trisaccharide verwendet werden, wie beispielsweise Lactose, Maltose, Saccharose, Trehalose, Gentianose, Kestose und Raffinose. In einer besonders bevorzugten Ausführungsform der Erfindung handelt es sich bei dem Kohlenhydrat um eine Substanz ausgewählt aus der Gruppe bestehend aus Glucose, Lactose, Maltose, Mannose und Saccharose.

Der Begriff "Zuckeralkohol", wie er in der vorliegenden Anmeldung verwendet wird, bezeichnet Monosaccharid-Zuckeralkohole der allgemeinen Summenformel CₙH₂ₙ₊₂Oₙ und Disaccharid-Alkohole der allgemeinen Summenformel CₙH₂ₙOₙ₋₁, welche jeweils natürlichen oder synthetischen Ursprungs sein können. Bevorzugte Monosaccharid-Zuckeralkohole umfassen Glycerol, Erythritol, Threitol, Ribitol, Arabinitol, Xylitol, Allitol, Altritol, Galactitol, Glucitol, Iditol und Mannitol, die jeweils in der D-Form oder in der L-Form vorliegen können. Als Disaccharid-Zuckeralkohole können insbesondere Isomalt, Lactitol und Maltitol verwendet werden. In einer besonders bevorzugten Ausführungsform der Erfindung handelt es sich bei dem Zuckeralkohol um eine Substanz ausgewählt aus der Gruppe bestehend aus Glucitol, Glycerol, Lacitol, Mannitol und Xylitol.

Nach Aufnahme der Probe kann das Probennahmeelement, vorzugsweise durch leichtes mechanisches Aufdrücken seiner Probennahmefläche(n), mit einem Bereich des Testelements in Kontakt gebracht werden, welcher zur Applikation der den Analyten enthaltenden Probe ausgebildet ist, wobei zumindest ein Teil der Probe von dem Probennahmeelement auf das Testelement übertragen wird. Der Druck, mit dem das Probennahmeelement auf das Testelement aufgedrückt wird, sollte dabei mindestens so groß sein, dass ein flächiger Kontakt zwischen der Oberfläche des Testelements und der Probennahmefläche/den Probennahmeflächen des Probennahmeelements besteht und insofern eine fluide Kommunikation zwischen beiden Elementen ermöglicht wird.

In einer bevorzugten Variante des erfindungsgemäßen Verfahrens führt das Inkontaktbringen von Probennahmeelement und Testelement zu einem direkten Kontakt zwischen der Probe (bzw. dem Analyten) und dem auf dem Testelement befindlichen analytbindenden Rezeptormolekül. Hierdurch wird ein schneller Kontakt zwischen dem Analyten und dem analytbindenden Rezeptormolekül realisiert, wodurch eine rasche Bildung des Komplexes aus Analyt und analytbindendem Rezeptormolekül erfolgen kann und die Sensitivität und Spezifität des Nachweisverfahrens signifikant verbessert wird. Ungeachtet dessen sieht vorliegende Erfindung auch die Möglichkeit vor, dass ein Kontakt zwischen dem Analyten und dem analytbindenden Rezeptormolekül erst nach Übertragung der Probe vom Probennahmeelement auf das Testelement und dem Start der Chromatographie stattfindet.

Um die Sensitivität und Spezifität des Verfahrens weiter zu verbessern, umfasst das Testelement in einer bevorzugten Variante zusätzlich mindestens ein Mittel, welches beispielsweise durch Blockierung bzw. Zerstörung unspezifischer Bindungsstellen in der Probe oder/und durch Veränderung der Probenkonsistenz eine chemische oder/und mechanische Aufbereitung der den Analyten enthaltenden Probe bewirkt. Auf diese Weise steht der Analyt optimal für eine Bindung an das analytbindende Rezeptormolekül zur Verfügung, womit die Zugänglichkeit des Analyten für das analytbindende Rezeptormolekül sowie der Transport über die einzelnen Bereiche des Testelements verbessert wird.

Sofern ein chemisches Probenaufbereitungsmittel eingesetzt wird, so kann dieses beispielsweise auf dem Testelement imprägniert sein, wobei zur Imprägnierung vorzugsweise eine wässrige oder nicht-wässrige Lösung, welche das chemische Probenaufbereitungsmittel in einer Konzentration von etwa 0.01 bis etwa 5 Gew.-% enthält, eingesetzt wird. Besonders bevorzugt gelangen im Testelement mindestens ein chemisches Probenaufbereitungsmittel und mindestens ein mechanisches Probenaufbereitungsmittel parallel zur Anwendung.

Chemische Probenaufbereitungsmittel, welche im Rahmen des erfindungsgemäßen Verfahrens Anwendung finden können, umfassen insbesondere Säuren, Basen, Puffer, organische Lösungsmittel und Detergenzien, wobei Basen als besonders bevorzugt gelten. Konkrete Beispiele für Säuren umfassen anorganische Säuren (z.B. Salzsäure) und organische Säuren (z.B. Essigsäure und Zitronensäure). Beispielhafte Basen umfassen insbesondere Alkali- und Erdalkalimetallhydroxide, wie etwa Natriumhydroxid und Calciumhydroxid, während als Puffer u.a. Calciumcarbonat, Tris, PBS, Phosphatpuffer, Boratpuffer, BICINE-Puffer und HEPES verwendet werden können. Beispiele für Detergenzien umfassen u.a. Octylglucosid, Cholamidopropansulfonat, Polidocanol, Polyalkylenglykolether (z.B. Brij^{®}, Synperonic^{®}) und Polysorbate (z.B. Tween^{®} 20, Tween^{®} 80). Beispielhafte organische Lösungsmittel umfassen insbesondere Dimethylsulfoxid, Ethanol, Glycerin, Isopropanol, Methanol, und Gemische hiervon.

Mechanische Probenaufbereitungsmittel, welche im Rahmen des erfindungsgemäßen Verfahrens Anwendung finden können, umfassen beispielsweise Gewebe oder/und Vliese, insbesondere Vliese, mittels welchen die Probe vor Inkubation des Analyten mit dem analytbindenden Rezeptormolekül filtriert bzw. aufgetrennt wird, so dass speziell feste und viskose Probenbestandteile (z.B. feste und viskose Speichelbestandteile), die das Nachweisverfahren negativ beeinflussen können, zurückgehalten werden. Konkrete Beispiele für Vliese, welche eine mechanische Aufbereitung der Probe bewirken können, umfassen beispielsweise die kommerziell erhältlichen Produkte Ahlstrom 8964, Whatman Rapid 24Q und Freudenberg FS 2216, sind jedoch nicht auf diese beschränkt.

Um dem Benutzer das Inkontaktbringen von Testelement und Probennahmeelement zu erleichtern, können in einer Ausführungsform der Erfindung ein oder mehrere Testelemente in einem Gehäuse untergebracht sein. Das Gehäuse weist hierbei vorzugsweise mindestens eine Aussparung auf, über welche das Probennahmeelement mit dem Testelement in Kontakt gebracht werden kann. Sofern das Probennahmeelement mehrere Probennahmeflächen aufweist, so ist es als bevorzugt anzusehen, dass das Gehäuse lediglich eine einzige Aussparung zur Aufnahme des Probennahmeelements enthält. Alternativ ist es indessen auch möglich, dass das Gehäuse eine der Anzahl an Probennahmeflächen entsprechende Anzahl an Aussparungen enthält. Die Aussparung(en) kann/können in beliebiger Form (zueinander) angeordnet sein und jede dem Fachmann geeignet erscheinende Dimension und Geometrie aufweisen, ist/sind jedoch üblicherweise an die Anordnung, die Dimension und die Geometrie der Probennahmefläche(n) des Probennahmeelements angepasst. Hierdurch ist es beispielsweise möglich, eine vorab definierte Zusammenführung von Probennahmeelement und Testelement zu erwirken, um auf diese Weise eine unsachgemäße Benutzung des Testsystems zu vermeiden.

In einer weiteren Ausführungsform kann das Gehäuse ferner eine Haltevorrichtung umfassen, welche eine reversible Befestigung des Probennahmeelements am Gehäuse gestattet, so dass letzteres beispielsweise zur Probennahme abgenommen und nach Probennahme wieder am Gehäuse angebracht werden kann. Hierbei kann durch Wahl einer geeigneten Position für die Haltevorrichtung das Probennahmeelement derart auf dem Gehäuse platziert werden, dass ein intensiver Kontakt zwischen Probennahmeelement bzw. dessen Probennahmefläche(n) und dem Testelement sichergestellt wird, was eine gute Übertragung des Analyten vom Probennahmeelement auf das das analytbindende Rezeptormolekül umfassende Testelement gewährleistet.

Im Rahmen der vorliegenden Erfindung werden das Testelement und das mit dem Analyten benetzte Probennahmeelement zum Zwecke einer hohen Sensitivität und Spezifität der Analytenbestimmung vorzugsweise für einen Zeitraum von mindestens 10 Sekunden miteinander in Kontakt gebracht, wobei das auf dem Testelement befindliche analytbindende Rezeptormolekül mit dem zu bestimmenden Analyten inkubiert werden kann und gegebenenfalls zusätzlich eine chemische oder/und mechanische Aufbereitung der den Analyten enthaltenden Probe erfolgt. Auf diese Weise wird sichergestellt, dass der Analyt einerseits möglichst vollständig aus der Probenmatrix des Probennahmeelements herausgelöst wird und andererseits nahezu quantitativ mit dem analytbindenden Rezeptormolekül abreagieren kann. Als vorteilhaft hat sich in diesem Zusammenhang eine Inkubationszeit von etwa 10 Sekunden bis etwa 600 Sekunden, stärker bevorzugt von etwa 20 Sekunden bis etwa 180 Sekunden, am stärksten bevorzugt von etwa 30 Sekunden bis etwa 90 Sekunden, erwiesen. Durch eine derartige Verfahrensführung kann das für die Bestimmung des Analyten benötigte Probenvolumen üblicherweise auf weniger als 10 µl reduziert und die Sensitivität des Testsytems deutlich verbessert werden.

Im Anschluss an das Aufbringen der den Analyten enthaltenden Probe auf das Testelement wird das Testelement mit einem Elutionsmittel in Kontakt gebracht. Zu diesem Zweck umfasst das Testelement vorzugsweise einen (endständigen) Bereich, welcher zur Aufnahme von Elutionsmittel ausgebildet ist und üblicherweise ein saugfähiges Material, wie beispielsweise Gewebe oder/und Vlies, umfasst. Nach Benetzung dieses Bereichs mit Elutionsmittel wandert das Elutionsmittel durch die verschiedenen Bereiche des Testelements, wobei Analyt, analytbindendes Rezeptormolekül sowie deren Komplexe entsprechend mittransportiert werden. Hierbei kann vorteilhafterweise die Kapillarwirkung der einzelnen Komponenten des Testelements ausgenutzt werden, welche derart angeordnet bzw. miteinander verbunden sind, dass ein ununterbrochener Elutionsmittelfluss gewährleistet ist.

Als Elutionsmittel kann im Rahmen der vorliegenden Erfindung grundsätzlich jedes dem Fachmann als geeignet erscheinende Elutionsmittel verwendet werden. Vorzugsweise gelangen in dem hierin beschriebenen Verfahren allerdings Wasser und wässrige Pufferlösungen zur Anwendung, welche gegebenenfalls weitere Substanzen wie beispielsweise ein Kohlenhydrat, einen Zuckeralkohol, ein Detergenz, ein Salz oder/und ein organisches Lösungsmittel, wie jeweils oben beschrieben, in einer Konzentration von üblicherweise etwa 0.05 bis etwa 1.5 Gew.-% umfassen können. Besonders bevorzugt gelangt im Rahmen des erfindungsgemäßen Verfahrens ein wässriges Elutionsmittel zum Einsatz, welches Magnesiumsulfat und eine Kombination von Boraten (z.B. Magnesium-Chlor-Borat, Natriumtetraborat, Calciumborat und Calcium-Natrium-Borat) als Bestandteile umfasst, durch dessen Verwendung eine Erhöhung der Sensitivität oder/und Spezifität der Analytenbestimmung erzielt werden kann.

Sofern das Testelement in ein Gehäuse eingebracht ist, so bestehen in Abhängigkeit von der Ausgestaltung des Gehäuses verschiedene Möglichkeiten, eine Benetzung des Testelements mit Elutionsmittel zu erwirken. Ist das Testelement etwa vollständig in dem Gehäuse gelagert, so kann das Elutionsmittel beispielsweise durch Aufdrücken auf eine das Elutionsmittel enthaltende Ampulle, welche vorzugsweise innerhalb des Gehäuses gelagert ist, auf den zur Aufnahme von Elutionsmittel ausgebildeten Bereich des Testelements aufgebracht werden. Ragt der zur Aufnahme von Elutionsmittel ausgebildete Bereich des Testelements indessen aus dem Gehäuse heraus, so besteht die Möglichkeit, den Bereich in das Elutionsmittel einzutauchen.

Nach Inkontaktbringen des Testelements mit dem Elutionsmittel wird die Bestimmung des Analyten in Gang gesetzt, welche beispielsweise ein kompetitives Testformat oder/und ein nicht-kompetitives Testformat (Sandwich-Testformat) umfassen kann. Besonders bevorzugt ist erfindungsgemäß eine Kombination aus einem kompetitiven Testformat und einem nicht-kompetitiven Testformat. Üblicherweise umfasst das erfindungsgemäße Nachweisverfahren zunächst die Bildung eines Komplexes aus Analytmolekülen und analytbindendem, gegebenenfalls markiertem Rezeptormolekül, welcher mit Hilfe des Elutionsmittels, beispielsweise zusammen mit ungebundenen Rezeptormolekülen oder/und weiteren in der Probe vorhandenen Substanzen, bis zu einem Bereich des Testelements weitertransportiert wird, der zur Detektion des Analyten ausgebildet ist.

Der zur Detektion, insbesondere zur optischen Detektion des Analyten ausgebildete Bereich umfasst üblicherweise mehrere definierte Abschnitte, in welchen unterschiedliche Reagenzien immobilisiert sein können. In einer Ausführungsform umfasst dieser Bereich einen Abschnitt, welcher zur Bindung von ungebundenen analytbindenden Rezeptormolekülen ausgebildet ist, einen Abschnitt, welcher zur Bindung des Komplexes aus Analyt und analytbindendem Rezeptormolekül ausgebildet ist, und gegebenenfalls einen Abschnitt, in welchem analytenunabhängig ein Kontrollsignal erzeugt wird. Der zur Detektion des Analyten ausgebildete Bereich kann dabei aus einem oder mehreren Materialien gebildet sein, welche dem Fachmann für die Zwecke der Erfindung geeignet erscheinen, wie beispielsweise Membranen aus Nylon^{®}, Nitrocellulose oder Polyvinylidenfluorid.

Im kompetitiven Testformat kann der zur Bindung von ungebundenen analytbindenden Rezeptormolekülen vorgesehene Abschnitt beispielsweise immobilisierte Analytanaloga, insbesondere Polyhaptene, umfassen, welche die analytbindenden, gegebenenfalls markierten Rezeptormoleküle infolge Ausbildung eines Komplexes an einer definierten Position auf dem Testelement (Abfanglinie) abfangen und auf diese Weise die Erzeugung falsch-positiver Ergebnisse vermeiden. Der Komplex aus Analyt und analytbindendem Rezeptormolekül wird an der Abfanglinie üblicherweise nicht immobilisiert, da der Analyt die hierzu erforderlichen Bindungsstellen am analytbindenden Rezeptormolekül blockiert.

Der zur Bindung des Komplexes aus Analyt und analytbindendem Rezeptormolekül ausgebildete Abschnitt umfasst vorzugsweise einen für das analytbindende Rezeptormolekül spezifischen Bindungspartner (z.B. einen Antikörper), welcher eine Immobilisierung des Komplexes aus Analyt und analytbindendem Rezeptormolekül an einer vorbestimmten Position auf dem Testelement (Testlinie) bewirkt und eine optische Bestimmung des Analyten ermöglicht, sofern das analytbindende Rezeptormolekül eine optische Markierung trägt. Die Immobilisierung des Komplexes erfolgt dabei in der Regel über eine freie Bindungsstelle des analytbindenden Rezeptormoleküls, wobei eine Färbung der Testlinie mit einem positiven Nachweis des Analyten einhergeht.

Um das Signal an der Testlinie eindeutig ablesen zu können und Verwechslungen mit der Abfanglinie auszuschließen, kann die Abfanglinie gegebenenfalls in geeigneter Weise abgedeckt werden. Sofern das Testelement darüber hinaus einen Kontrollabschnitt umfasst, erscheint bei Durchführung des Verfahrens zusätzlich eine Kontrolllinie, die als Indikator für eine einwandfreie Funktionalität des Testelements fungiert. Überschüssiges Elutionsmittel, welches den zur Detektion des Analyten ausgebildeten Bereich des Testelements verlässt, kann gegebenenfalls mit Hilfe eines flüssigkeitsabsorbierenden Materials in einem eigens hierfür ausgebildeten Bereich des Testelements aufgenommen werden.

Im nicht-kompetitiven Testformat bzw. Sandwich-Testformat umfasst der zur Detektion des Analyten ausgebildete Bereich keinen Abschnitt, welcher zur Bindung von ungebundenen analytbindenden Rezeptormolekül ausgebildet ist. In diesem Fall wird zur Erzeugung des Komplexes aus Analyt und analytbindendem Rezeptormolekül vorzugsweise ein spezieller analytspezifischer Bindungspartner eingesetzt, wie er beispielsweise in EP 1 579 222 B1 beschrieben ist. Derartige Komplexe können in der Folge mittels eines komplexspezifischen Bindungspartners immobilisiert werden, was letztlich zu einer vereinfachten Detektion des Analyten führt, da die Erzeugung falsch-positiver Signale, welche u.a. durch nicht an der Abfanglinie gebundene analytbindende Rezeptormoleküle bewirkt werden können, vermieden wird.

Der zur Bindung des Komplexes aus Analyt und analytbindendem Rezeptormolekül ausgebildete Abschnitt umfasst dann vorzugsweise einen komplexspezifischen Bindungspartner (z.B. einen Antikörper), welcher eine Immobilisierung des Komplexes aus Analyt und analytbindendem Rezeptormolekül an einer vorbestimmten Position auf dem Testelement (Testlinie) bewirkt und auf diese Weise eine optische Bestimmung des Analyten ermöglicht, sofern das analytbindende Rezeptormolekül eine optische Markierung trägt. Die Immobilisierung des Komplexes erfolgt dabei in der Regel über eine freie Bindungsstelle des komplexspezifischen Bindungspartners, wobei eine Färbung der Testlinie mit einem positiven Nachweis des Analyten einhergeht.

Die im letzten Schritt des erfindungsgemäßen Verfahrens durchgeführte qualitative oder/und quantitative Bestimmung des Analyten kann auf beliebige Art und Weise erfolgen. Hierzu können grundsätzlich alle aus dem Stand der Technik bekannten Methoden zum Nachweis einer Komplexbildung eingesetzt werden, welche ein messbares Signal erzeugen, das manuell oder unter Verwendung geeigneter Mittel ausgewertet bzw. ausgelesen werden kann. Im Rahmen der vorliegenden Erfindung gelangen bevorzugt optische Nachweismethoden, insbesondere photometrische oder fluorimetrische Nachweismethoden, zum Einsatz. Besonders bevorzugt ist erfindungsgemäß ein optisch-visueller Nachweis des Analyten.

Besonders bevorzugt werden mittels des erfindungsgemäßen Verfahrens mehrere unterschiedliche Analyten, insbesondere 5 bis 50 unterschiedliche Analyten, gleichzeitig bestimmt. In diesem Fall ist es bevorzugt, dass der gleichzeitige Nachweis mehrerer unterschiedlicher Analyten aus einer Substanzklasse, wie beispielsweise der gleichzeitige Nachweis verschiedener Cannabinoide, über ein einziges analytbindendes Rezeptormolekül erfolgt. Sofern mittels des Testelements Analyten aus unterschiedlichen Substanzklassen nachgewiesen werden sollen, kann der zur Applikation der Probe ausgebildete Bereich insbesondere eine der Anzahl an unterschiedlichen Substanzklassen entsprechende Anzahl an unterschiedlichen analytbindenden Rezeptormolekülen und, sofern das Testelement keinen Abschnitt zum Abfangen von ungebundenen analytbindenden Rezeptormolekülen enthält, gegebenenfalls eine der Anzahl an unterschiedlichen Substanzklassen entsprechende Anzahl an komplexspezifischen Bindungspartnern, wie sie vorstehend definiert sind, umfassen. Auf diese Weise kann sichergestellt werden, dass die parallele Bestimmung von Analyten aus unterschiedlichen Substanzklassen im Wesentlichen unabhängig voneinander erfolgt und keinerlei Interferenzen auftreten.

Das erfindungsgemäße Verfahren ermöglicht die Bestimmung eines oder mehrerer Analyten mit hoher Sensitivität und Spezifität. So ist es erfindungsgemäß bevorzugt, Analyten mit einer Spezifität von mindestens 95% oder/und einer Sensitivität von mindestens 90% zu bestimmen. Stärker bevorzugt erfolgt die Bestimmung mit einer Spezifität von mindestens 98% oder/und einer Sensitivität von mindestens 95%, so dass mittels des hierin beschriebenen Verfahrens Analyten bis hinab zu einer unteren Nachweisgrenze von etwa 1 ng/ml Probe nachgewiesen werden können. Entsprechend den SAMHSA-Vorgaben (Department of Health and Human Services: Proposed revisions to mandatory guidelines for federal workplace drug testing programs, Federal Register (2004), 69, 19673-19732) bzw. Bosker et al. (Clin. Chem. (2009), 55, 1910-1931) soll die untere Nachweisgrenze für Δ⁹-Tetrahydrocannabinol bei 4 ng/ml Speichel, die untere Nachweisgrenze für synthetische Cannabinoide bei 10 ng/ml Speichel liegen.

Das erfindungsgemäße Verfahren kann zur Bestimmung einer beliebigen biologischen oder chemischen Substanz verwendet werden, welche beispielsweise unter Verwendung immunologischer Techniken oder Rezeptor-Liganden-Techniken nachweisbar ist. Vorzugsweise wird das hierin beschriebene Verfahren indessen zum Nachweis von natürlichen, halbsynthetischen oder vollsynthetischen Betäubungsmitteln, wie sie im deutschen Betäubungsmittelgesetz aufgelistet sind und welche *in vivo* an ein erfindungsgemäß eingesetztes Rezeptormolekül binden, verwendet. Konkrete Beispiele für derartige Betäubungsmittel umfassen u.a. Dissoziativa, Delirantia, Empathogene, Entaktogene, Hypnotika, Narkotika, Psychedelika, Sedativa, und Stimulantia, sind jedoch nicht auf diese beschränkt.

Stärker bevorzugt wird erfindungsgemäß mindestens ein Analyt ausgewählt aus der Gruppe bestehend aus natürlichen, halbsynthetischen oder vollsynthetischen Amphetaminen, Benzodiazepinen, Cannabinoiden, Ketaminen, Methamphetaminen, Opioiden und Tropan-Alkaloiden bestimmt, wobei Cannabinoide und Opioide als Analyten bevorzugt sind. Innerhalb der Gruppe der Cannabinoide sind Δ⁹-Tetrahydrocannabinol und synthetische Cannabinoide als Analyten bevorzugt, während bevorzugte Beispiele für Opioide insbesondere Codein, Desomorphin, Fentanyl, Heroin, Methadon und Morphin umfassen. Besonders bevorzugt erfolgt erfindungsgemäß ein Nachweis von Δ⁹-Tetrahydrocannabinol und synthetischen Cannabinoiden.

Der Analyt kann aus einer beliebigen Quelle stammen, wie beispielsweise von einem mit dem Analyten benetzten Objekt, insbesondere von der Oberfläche eines mit dem Analyten benetzten Objekts, oder aus einem Körperfluid, insbesondere aus Blut, Urin, Speichel oder Schweiß. Bevorzugt wird mittels des hierin beschriebenen Verfahrens das Vorhandensein oder/und die Menge eines Analyten in einer Probe aus Speichel oder Schweiß bestimmt. Die zur Durchführung des Verfahrens benötigte Probenmenge beträgt üblicherweise von etwa 0.1 µl bis etwa 200 µl, bevorzugt von etwa 0.5 µl bis etwa 40 µl, stärker bevorzugt von etwa 1 µl bis 15 µl, und am stärksten bevorzugt von etwa 2 µl bis etwa 10 µl.

In einem weiteren Aspekt betrifft die Erfindung ein Testelement zur Bestimmung eines Analyten, umfassend:
(i) gegebenenfalls einen ersten Bereich, welcher zur Aufnahme von Elutionsmittel ausgebildet ist,
(ii) einen zweiten Bereich, welcher zur Applikation einer den Analyten enthaltenden Probe ausgebildet ist,
(iii) einen dritten Bereich, welcher zur Detektion, vorzugsweise zur optischen Detektion des Analyten ausgebildet ist,
(iv) gegebenenfalls einen vierten Bereich, welcher zur Aufnahme von überschüssigem Elutionsmittel ausgebildet ist, und
(v) gegebenenfalls ein Gehäuse,
wobei das Testelement mindestens ein den Analyten bindendes Rezeptormolekül umfasst, wobei die ligandenbindende Domäne des Rezeptormoleküls in nativer, verkürzter oder mutierter Form vorliegt und gegebenenfalls mit einem heterologen Molekül konjugiert ist.

In noch einem weiteren Aspekt betrifft die Erfindung ein Probennahmeelement zur Aufnahme eines Analyten von einem Objekt und zur Übertragung des Analyten auf ein Testelement, wobei das Probennahmeelement mindestens ein den Analyten bindendes Rezeptormolekül umfasst, wobei die ligandenbindende Domäne des Rezeptormoleküls in nativer, verkürzter oder mutierter Form vorliegt und gegebenenfalls mit einem heterologen Molekül konjugiert ist.

In noch einem weiteren Aspekt betrifft die Erfindung einen Kit zur Bestimmung eines Analyten, welcher vorzugsweise zur Durchführung des vorstehend beschriebenen Verfahrens verwendet wird und nachfolgende Bestandteile umfasst:
(a) ein Testelement, umfassend
   (i) gegebenenfalls einen ersten Bereich, welcher zur Aufnahme von Elutionsmittel ausgebildet ist,
   (ii) einen zweiten Bereich, welcher zur Applikation einer den Analyten enthaltenden Probe ausgebildet ist,
   (iii) einen dritten Bereich, welcher zur Detektion, vorzugsweise zur optischen Detektion des Analyten ausgebildet ist,
   (iv) gegebenenfalls einen vierten Bereich, welcher zur Aufnahme von überschüssigem Elutionsmittel ausgebildet ist, und
   (v) gegebenenfalls ein Gehäuse, und
(b) ein Probennahmeelement, welches zur Aufnahme einer den Analyten enthaltenden Probe von einer Oberfläche ausgebildet ist,
wobei das Testelement oder/und das Probennahmeelement mindestens ein den Analyten bindendes Rezeptormolekül umfasst, wobei die ligandenbindende Domäne des Rezeptormoleküls in nativer, verkürzter oder mutierter Form vorliegt und gegebenenfalls mit einem heterologen Molekül konjugiert ist.

Was bevorzugte Ausgestaltungen des erfindungsgemäßen Testelements, des erfindungsgemäßen Probennahmeelements und des in dem erfindungsgemäßen Kit enthaltenen Testelements bzw. Probennahmeelements betrifft, so wird auf die Ausführungen in Zusammenhang mit der Beschreibung des erfindungsgemäßen Verfahrens verwiesen.

### Beschreibung der Figuren

- **Figur 1:**: Chemische Struktur von Δ⁹-Tetrahydrocannabinol und verschiedenen ausgewählten Substanzen aus der Gruppe der synthetischen Cannabinoide.

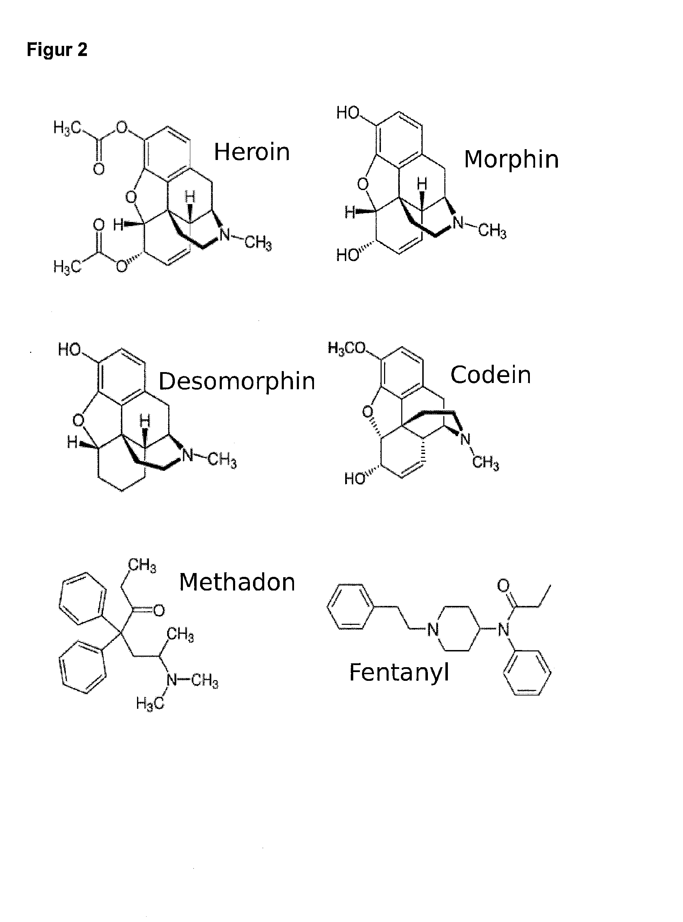

- **Figur 2:**: Chemische Struktur von verschiedenen ausgewählten Substanzen aus der Gruppe der Opioide.

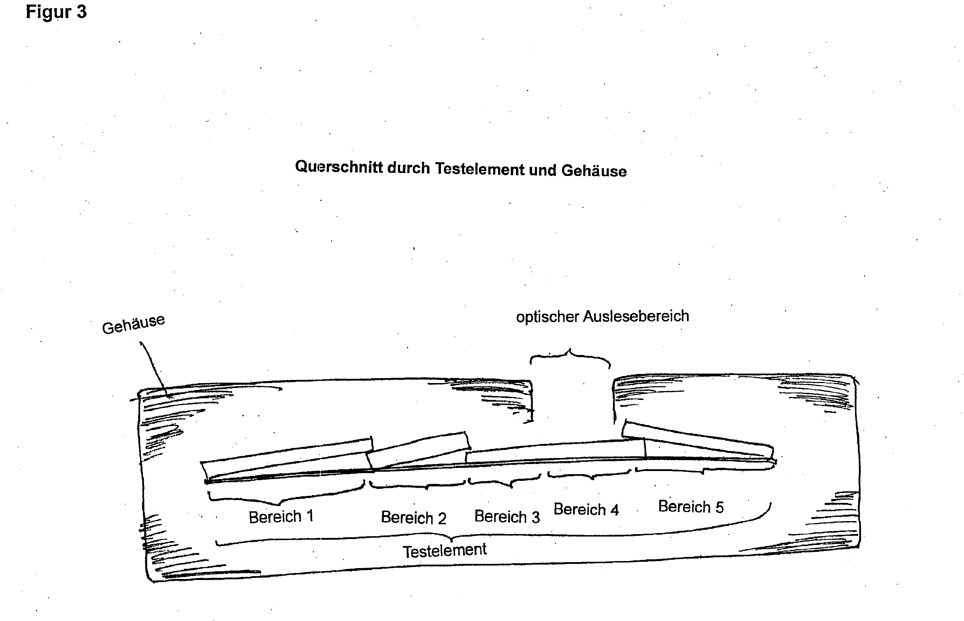

- **Figur 3:**: Querschnitt einer Ausführungsform eines Testelements zur Durchführung des Verfahrens gemäß der vorliegenden Erfindung. Das Testelement umfasst:
(a) einen ersten Bereich, welcher zur Aufnahme von Elutionsmittel ausgebildet ist,
(b) einen zweiten Bereich, welcher zur Applikation einer den Analyten enthaltenden Probe ausgebildet ist und ein analytbindendes Rezeptormolekül sowie ein Analyt-Übertragungsreagenz enthält,
(c) einen dritten Bereich, welcher zur Bindung von ungebundenem analytbindendem Rezeptormolekül ausgebildet ist und Polyhaptene umfasst,
(d) einen vierten Bereich, welcher zur optischen Detektion des Analyten ausgebildet ist und eine Testlinie umfasst, sofern ein Komplex aus Analyt und analytbindendem Rezeptormolekül gebildet worden ist,
(e) einen fünften Bereich, welcher zur Aufnahme von überschüssigem Elutionsmittel ausgebildet ist, und
(f) ein Gehäuse.

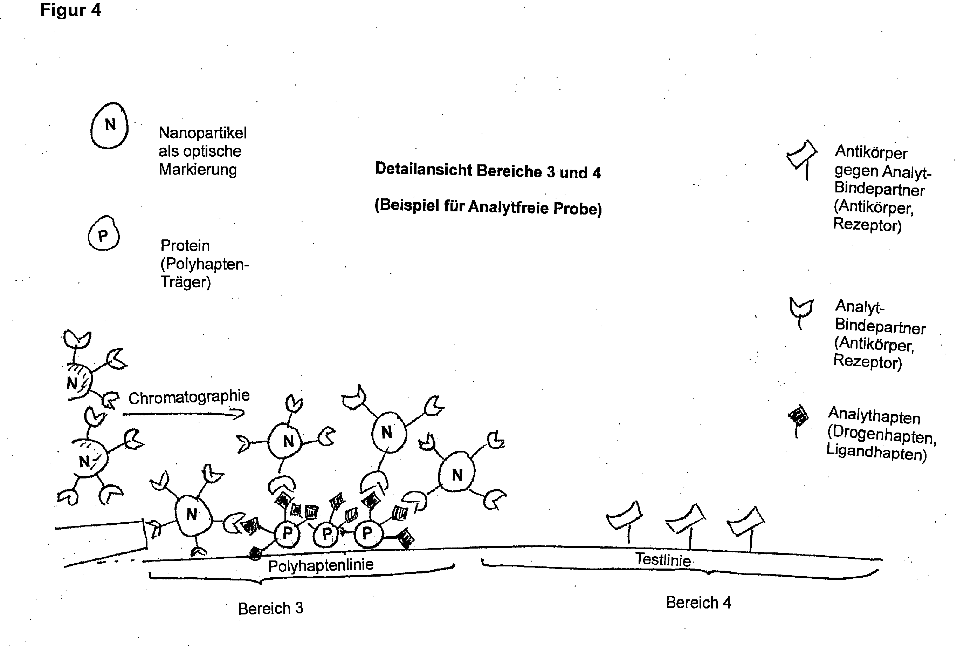

- **Figur 4:**: Detailansicht der Bereiche 3 und 4 des Testelements gemäß Figur 3, in welcher der Chromatographieverlauf einer Probe ohne Analyt dargestellt ist. Das analytbindende Rezeptormolekül, welches an die Oberfläche geeigneter Nanopartikel gebunden ist, wird in Bereich 3 des Testelements an der Polyhaptenlinie immobilisiert, da es über seine freien Bindungsstellen mit den Polyhaptenen wechselwirkt.

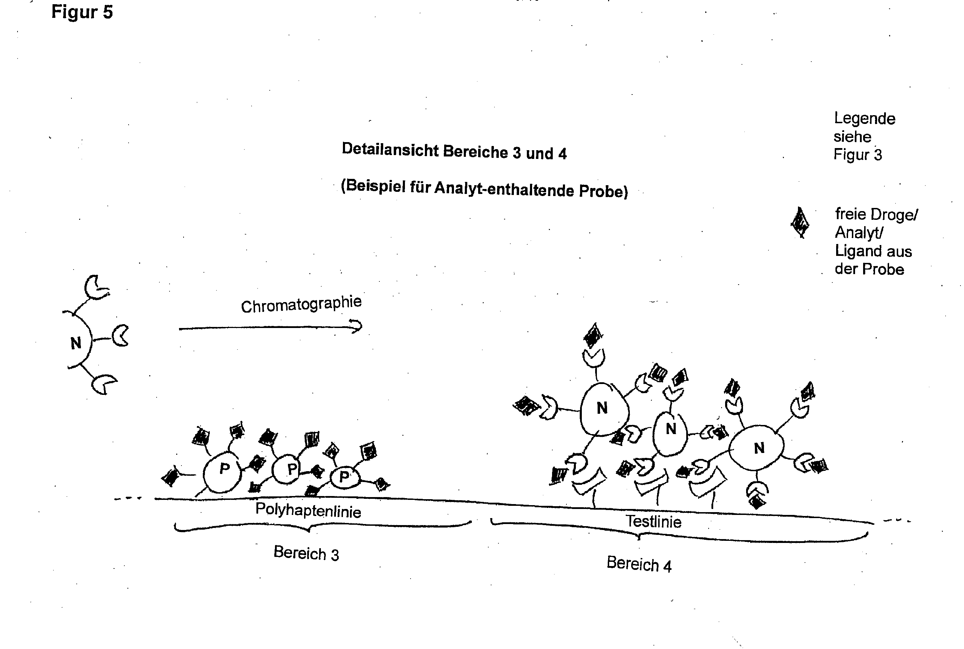

- **Figur 5:**: Detailansicht der Bereiche 3 und 4 des Testelements gemäß Figur 3, in welcher der Chromatographie-Verlauf einer Probe mit Analyt dargestellt ist. Das analytbindende Rezeptormolekül, welches an die Oberfläche geeigneter Nanopartikel gebunden ist, wird in Bereich 3 des Testelements nicht an der Polyhaptenlinie immobilisiert, da seine Bindungsstellen mit dem Analyten besetzt sind und keine Wechselwirkung mit den Polyhaptenen möglich ist. Stattdessen wandert der Komplex aus Analyt und analytbindendem Rezeptormolekül bis in den Bereich 4 des Testelements weiter, wo er an der Testlinie immobilisiert wird.
- **Figur 6:**: Nukleotidsequenz von humanem Cannabinoidrezeptor 1 (SEQ ID NO:1).

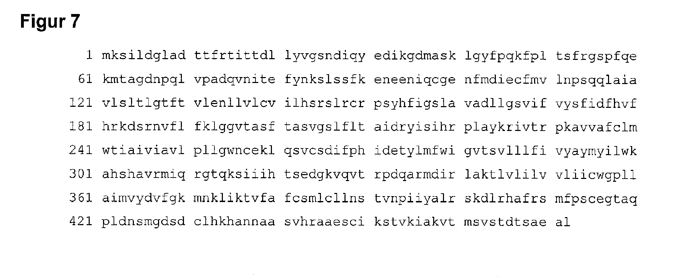

- **Figur 7:**: Aminosäuresequenz von humanem Cannabinoidrezeptor 1 (SEQ ID NO:2).

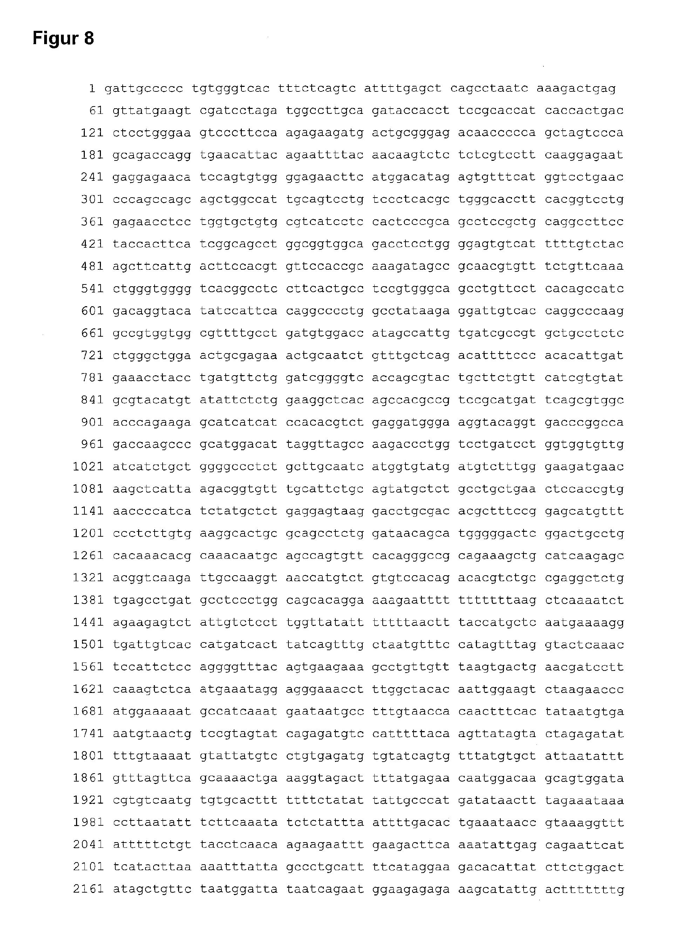

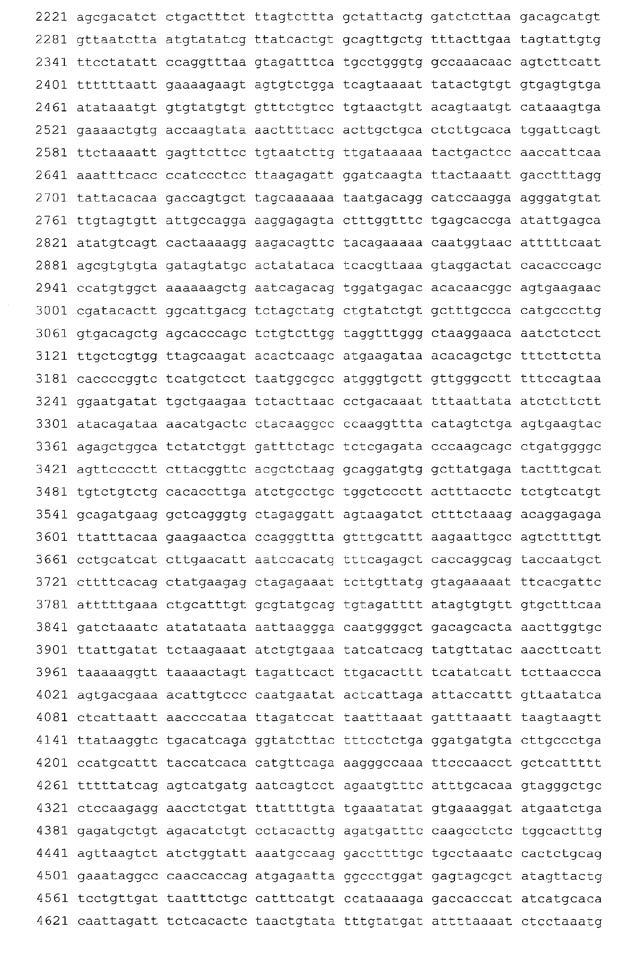

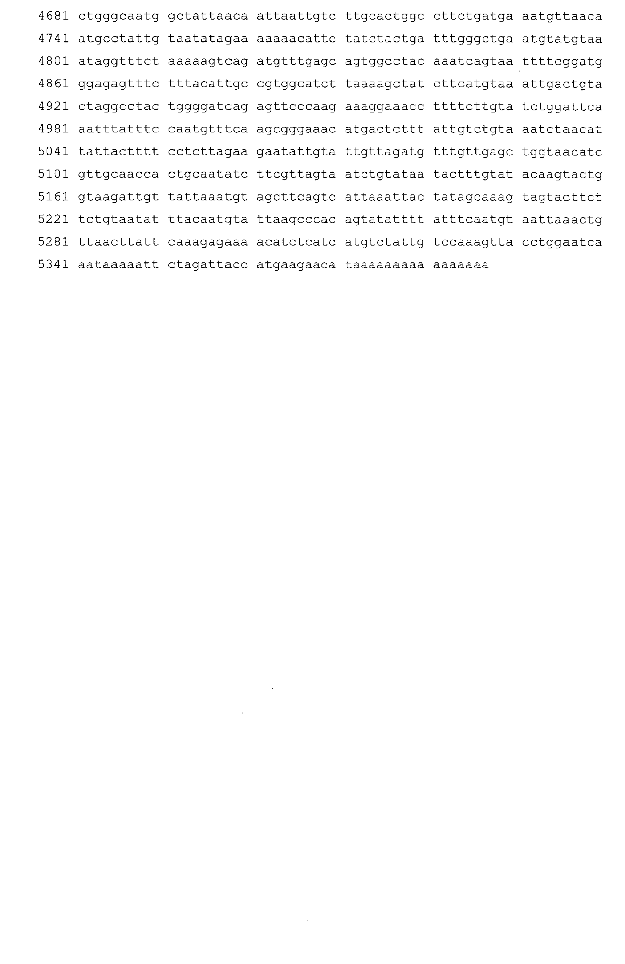

- **Figur 8:**: Nukleotidsequenz von humanem Cannabinoidrezeptor 2 (SEQ ID NO:3).

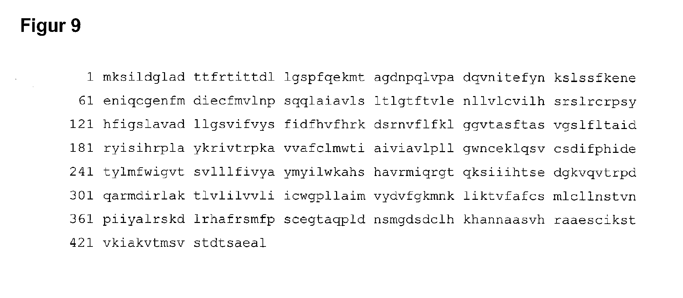

- **Figur 9:**: Aminosäuresequenz von humanem Cannabinoidrezeptor 2 (SEQ ID NO:4).

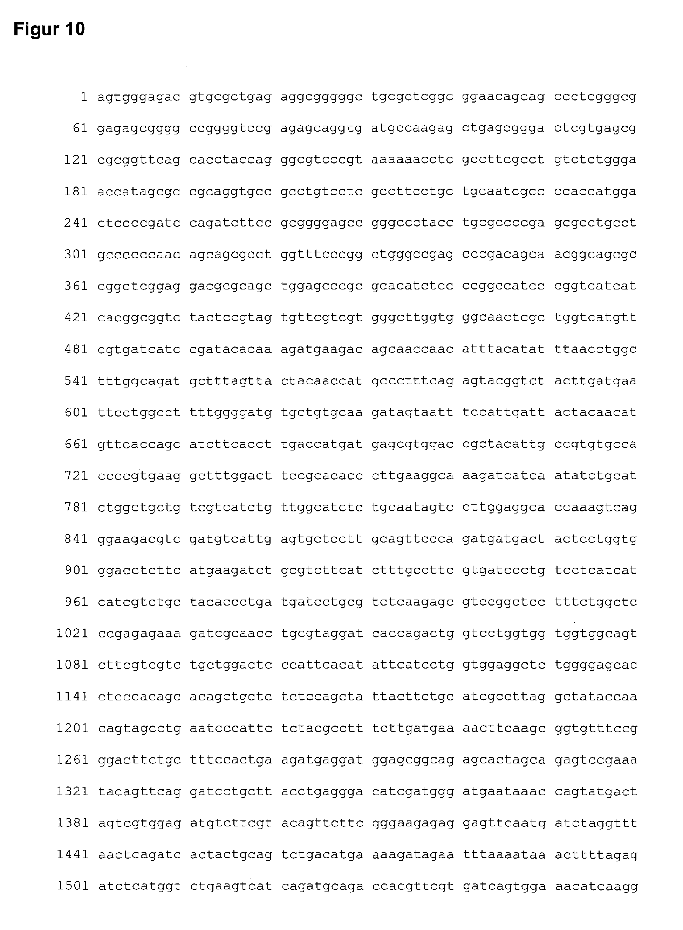

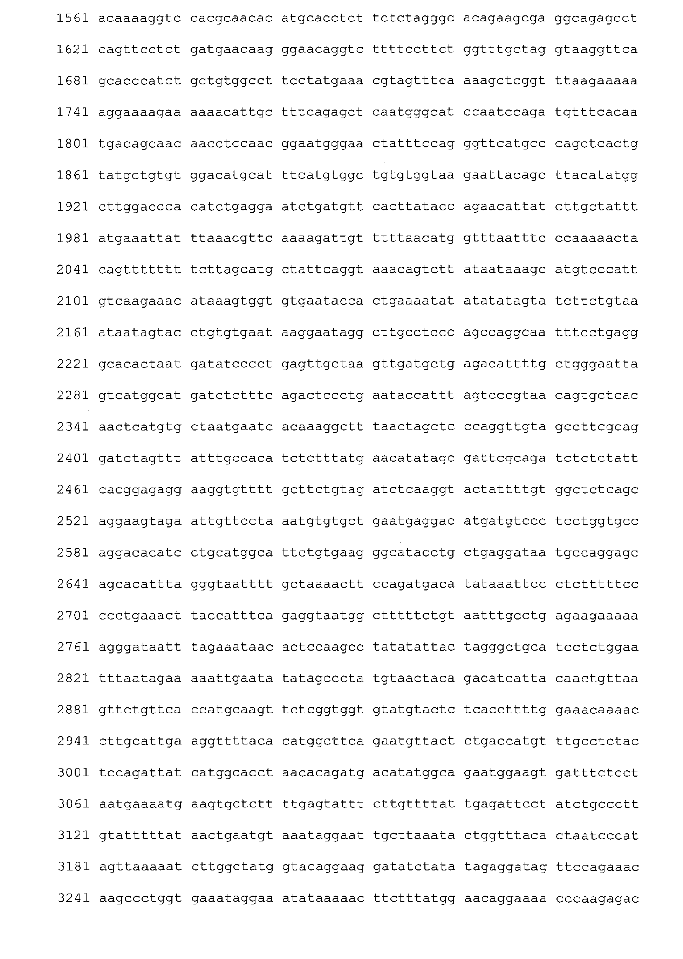

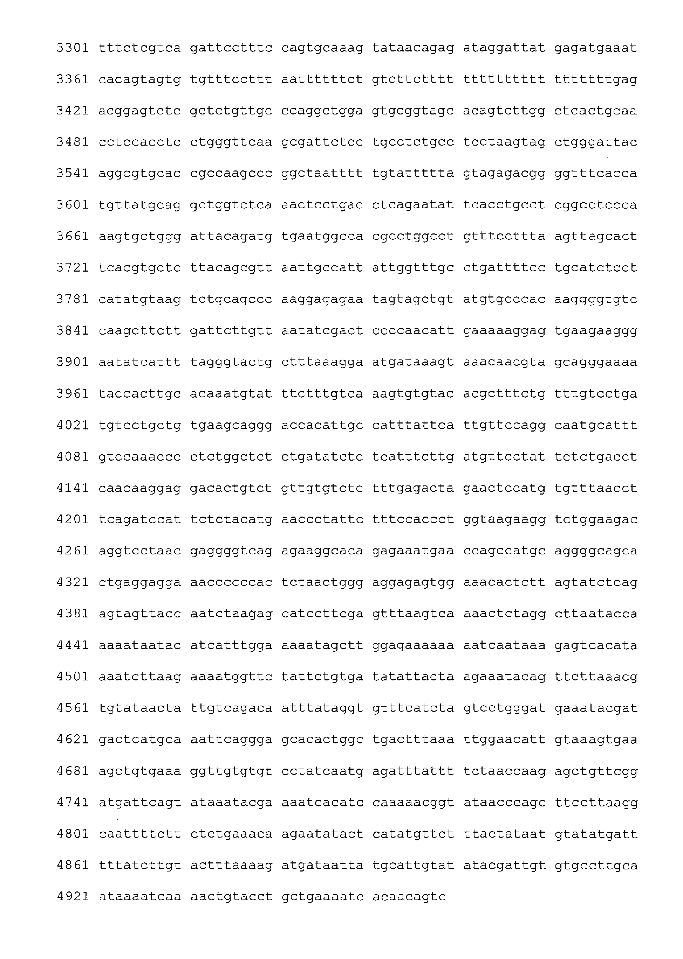

- **Figur 10:**: Nukleotidsequenz von humanem Opioidrezeptor κ1 (SEQ ID NO:5).

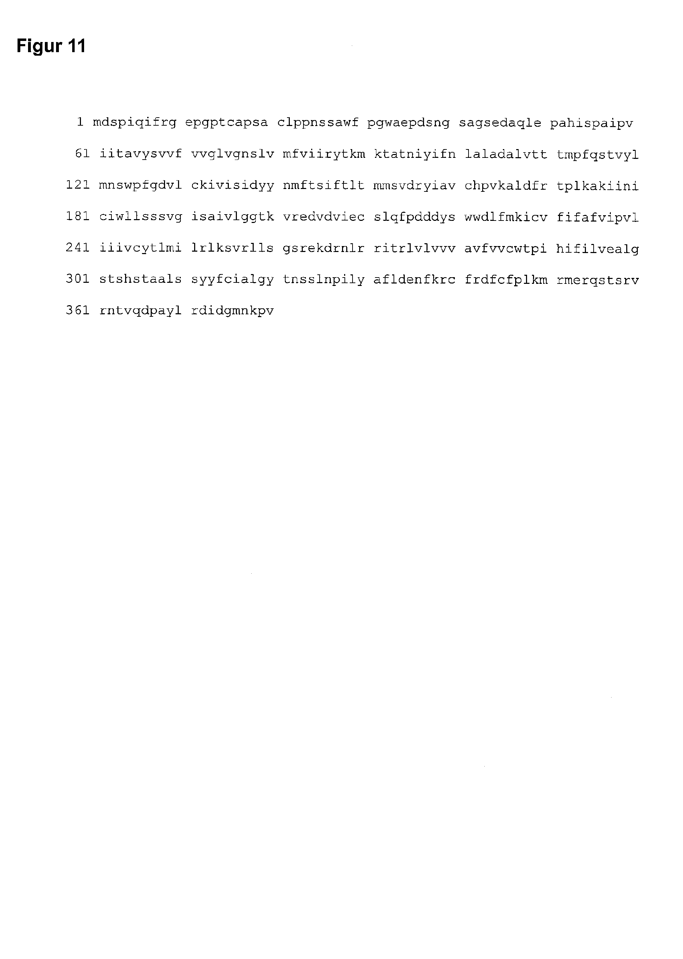

- **Figur 11:**: Aminosäuresequenz von humanem Opioidrezeptor κ1 (SEQ ID NO:6).

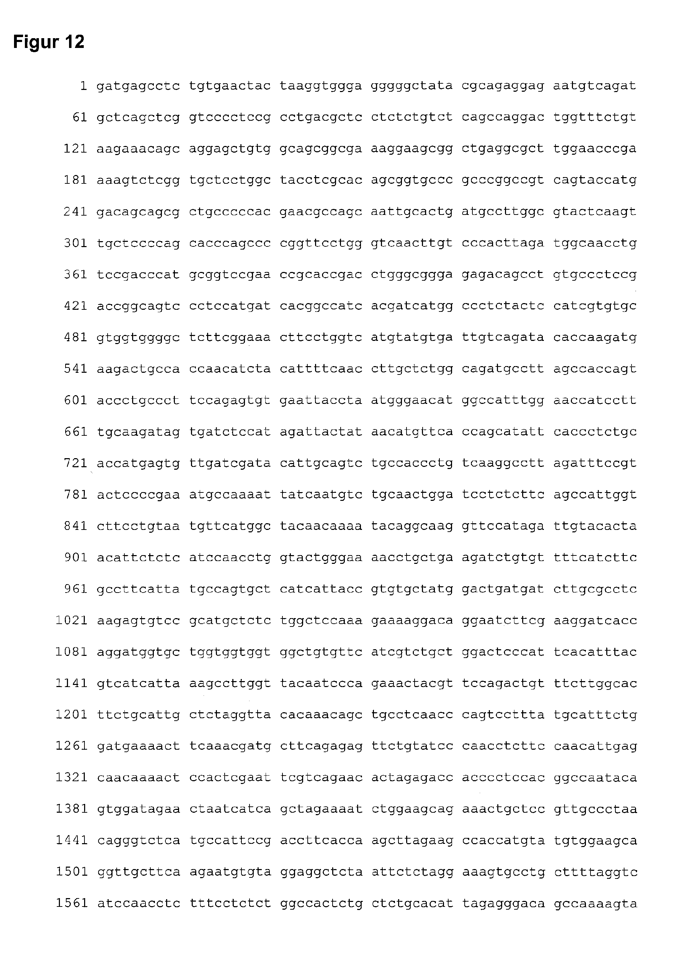

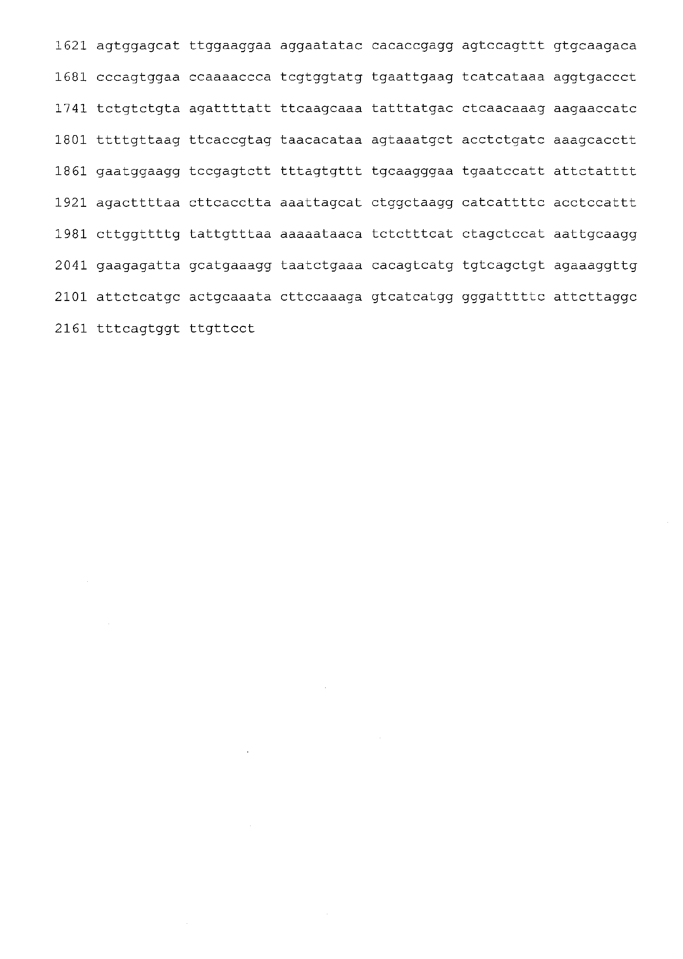

- **Figur 12:**: Nukleotidsequenz von humanem Opioidrezeptor µ1, Transkriptvariante MOR-1 (SEQ ID NO:7).

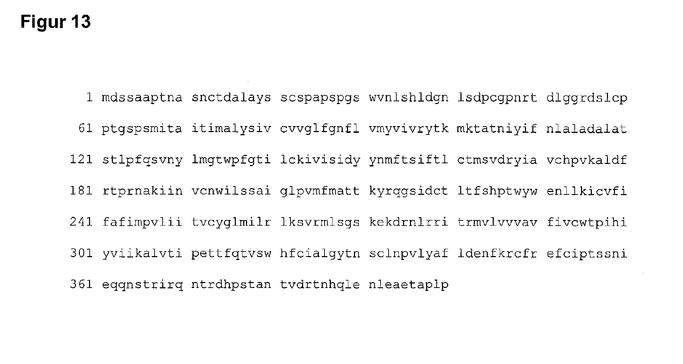

- **Figur 13:**: Aminosäuresequenz von humanem Opioidrezeptor µ1, Transkriptvariante MOR-1 (SEQ ID NO:8).

## Patentansprüche

1. Verfahren zur Bestimmung eines Analyten in einer Probe, umfassend die Schritte:
(a) Bereitstellen eines Testelements,
(b) Aufbringen der Probe auf das Testelement und
(c) Bestimmen des Vorhandenseins oder/und der Menge des Analyten auf dem Testelement,
**dadurch gekennzeichnet,**
**dass** das Testelement mindestens ein den Analyten bindendes Rezeptormolekül umfasst, wobei die ligandenbindende Domäne des Rezeptormoleküls in nativer, verkürzter oder mutierter Form vorliegt und gegebenenfalls mit einem heterologen Molekül konjugiert ist.

2. Verfahren zur Bestimmung eines Analyten in einer Probe, umfassend die Schritte:
(a) Bereitstellen eines Testelements,
(b) Aufnehmen der Probe von einer Oberfläche mit einem Probennahmeelement, wobei das Probennahmeelement eine oder mehrere Probennahmeflächen aufweist,
(c) Inkontaktbringen des Probennahmeelements mit dem Testelement, wobei zumindest ein Teil der Probe von dem Probennahmeelement auf das Testelement übertragen wird, und
(d) Bestimmen des Vorhandenseins oder/und der Menge des auf das Testelement übertragenen Analyten,
**dadurch gekennzeichnet,**
**dass** das Testelement mindestens ein den Analyten bindendes Rezeptormolekül umfasst, wobei die ligandenbindende Domäne des Rezeptormoleküls in nativer, verkürzter oder mutierter Form vorliegt und gegebenenfalls mit einem heterologen Molekül konjugiert ist.

3. Verfahren nach einem der Ansprüche 1 bis 2,
**dadurch gekennzeichnet,**
**dass** das Rezeptormolekül in einer löslichen Form oder immobilisiert auf dem Testelement vorliegt.

4. Verfahren nach einem der Ansprüche 1 bis 3,
**dadurch gekennzeichnet,**
**dass** ein Testelement zur Durchführung eines heterogenen, homogenen oder chromatographischen Tests, eines ELISA oder eines FRET-Tests verwendet wird, wobei das Testelement gegebenenfalls mikrofluidische Strukturen enthält und vorzugsweise ein chromatographischer Teststreifen ist.

5. Verfahren nach einem der Ansprüche 1 bis 4,
**dadurch gekennzeichnet,**
**dass** das Rezeptormolekül die ligandenbindende Domäne eines Betäubungsmittel-bindenden Rezeptormoleküls, insbesondere die ligandenbindende Domäne eines Cannabinoid- oder Opioid-bindenden Rezeptormoleküls umfasst.

6. Verfahren nach einem der Ansprüche 1 bis 5,
**dadurch gekennzeichnet,**
**dass** das Rezeptormolekül die ligandenbindende Domäne eines Rezeptormoleküls ausgewählt aus der Gruppe bestehend aus Cannabinoidrezeptor 1, Cannabinoidrezeptor 2, Opioidrezeptor ö, Opioidrezeptor κ, Opioidrezeptor µ1 und Opioidrezeptor µ2 umfasst.

7. Verfahren nach einem der Ansprüche 1 bis 6,
**dadurch gekennzeichnet,**
**dass** das Rezeptormolekül, insbesondere dessen ligandenbindende Domäne, eine verkürzte Aminosäuresequenz, insbesondere eine Verkürzung der Aminosäuresequenz um bis zu 10%, 20%, 30%, 40% oder 50% gegenüber der nativen Aminosäuresequenz, besitzt, wobei die Verkürzung am N-Terminus, am C-Terminus und/oder innerhalb der Proteinsequenz ist.

8. Verfahren nach einem der Ansprüche 1 bis 7,
**dadurch gekennzeichnet,**
**dass** das Rezeptormolekül, insbesondere dessen ligandenbindende Domäne, eine mutierte Aminosäuresequenz besitzt, welche zu mindestens 80%, insbesondere zu mindestens 95% identisch ist mit der Aminosäuresequenz der ligandenbindenden Domäne des nativen Rezeptormoleküls.

9. Verfahren nach einem der Ansprüche 1 bis 8,
**dadurch gekennzeichnet,**
**dass** das native Rezeptormolekül aus einem Säuger, insbesondere aus einem Menschen, stammt.

10. Verfahren nach einem der Ansprüche 1 bis 9,
**dadurch gekennzeichnet,**
**dass** das Rezeptormolekül mit einem heterologen Polypeptid, insbesondere mit einer Immunglobulindomäne konjugiert ist.

11. Verfahren nach einem der Ansprüche 1 bis 10,
**dadurch gekennzeichnet,**
**dass** die Probe
(a) ein Körperfluid, insbesondere Blut, Urin, Speichel oder Schweiß ist, oder
(b) von einem Objekt, insbesondere von der Oberfläche eines Objektes genommen wird.

12. Verfahren nach einem der Ansprüche 1 bis 11,
**dadurch gekennzeichnet,**
**dass** der Analyt ein natürliches, halbsynthetisches oder vollsynthetisches Betäubungsmittel, insbesondere ein Cannabinoid oder Opioid, ist, welches *in vivo* an das Rezeptormolekül bindet.

13. Verfahren nach einem der Ansprüche 1 bis 12,
**dadurch gekennzeichnet,**
**dass** mehrere Analyten, insbesondere 5 bis 50 unterschiedliche Analyten, gleichzeitig bestimmt werden, wobei insbesondere ein gemeinsamer Nachweis mehrerer unterschiedlicher Analyten über ein einziges Rezeptormolekül erfolgt.

14. Verfahren nach einem der Ansprüche 1 bis 13,
**dadurch gekennzeichnet,**
**dass** es eine Kombination aus einem kompetitiven Testformat und einem nicht-kompetitiven Testformat umfasst.

15. Testelement zur Bestimmung eines Analyten, umfassend:
(i) gegebenenfalls einen ersten Bereich, welcher zur Aufnahme von Elutionsmittel ausgebildet ist,
(ii) einen zweiten Bereich, welcher zur Applikation einer den Analyten enthaltenden Probe ausgebildet ist,
(iii) einen dritten Bereich, welcher zur Detektion, vorzugsweise zur optischen Detektion des Analyten ausgebildet ist,
(iv) gegebenenfalls einen vierten Bereich, welcher zur Aufnahme von überschüssigem Elutionsmittel ausgebildet ist, und
(v) gegebenenfalls ein Gehäuse,
**dadurch gekennzeichnet,**
**dass** es mindestens ein den Analyten bindendes Rezeptormolekül umfasst, wobei die ligandenbindende Domäne des Rezeptormoleküls in nativer, verkürzter oder mutierter Form vorliegt und gegebenenfalls mit einem heterologen Molekül konjugiert ist.

16. Probennahmeelement zur Aufnahme eines Analyten von einem Objekt und zur Übertragung des Analyten auf ein Testelement,
**dadurch gekennzeichnet,**
**dass** es mindestens ein den Analyten bindendes Rezeptormolekül umfasst, wobei die ligandenbindende Domäne des Rezeptormoleküls in nativer, verkürzter oder mutierter Form vorliegt und gegebenenfalls mit einem heterologen Molekül konjugiert ist.

17. Kit zur Bestimmung eines Analyten, umfassend:
(a) ein Testelement, umfassend
(i) gegebenenfalls einen ersten Bereich, welcher zur Aufnahme von Elutionsmittel ausgebildet ist,
(ii) einen zweiten Bereich, welcher zur Applikation einer den Analyten enthaltenden Probe ausgebildet ist,
(iii) einen dritten Bereich, welcher zur Detektion, vorzugsweise zur optischen Detektion des Analyten ausgebildet ist,
(iv) gegebenenfalls einen vierten Bereich, welcher zur Aufnahme von überschüssigem Elutionsmittel ausgebildet ist, und
(v) gegebenenfalls ein Gehäuse, und
(b) ein Probennahmeelement, welches zur Aufnahme einer den Analyten enthaltenden Probe von einer Oberfläche ausgebildet ist,
**dadurch gekennzeichnet,**
**dass** das Testelement oder/und das Probennahmeelement mindestens ein den Analyten bindendes Rezeptormolekül umfasst, wobei die ligandenbindende Domäne des Rezeptormoleküls in nativer, verkürzter oder mutierter Form vorliegt und gegebenenfalls mit einem heterologen Molekül konjugiert ist.
